# EUROPEAN PATENT APPLICATION

(11) **EP 4 492 059 A2**
(43) Date of publication of application: **15.01.2025**
(21) Application number: 24201788.7
(22) Date of filing: 16.05.2019
(51) Int. Cl.: G01N 33/50

(54) **METHODS OF MODULATING ANTIGENICITY TO ENHANCE RECOGNITION BY T-CELLS**

(30) Priority: 16.05.2018 US 201862672382 P
(62) Divisional of application: 19802800.3
(71) Applicant: THE GENERAL HOSPITAL CORPORATION, Boston, MA 02114 (US)
(72) Inventor: COBBOLD, Mark, Winchester (US); BENES, Cyril, Boston (US); SHI, Feng, Winchester (US)
(74) Representative: Maiwald GmbH

(57) **Abstract**

The present invention provides methods and compositions for increasing tumor antigenicity by increasing the level of expression of phosphoneoantigens in cells (e.g., restoring the expression of a phosphoneoantigen on the surface of a cancer cell). The invention also provides methods and compositions for enhancing recognition of phosphoneoantigens by immune cells.

## Description

### Background of the Invention

Cancer is caused by the accumulation of genetic and epigenetic changes that lead uncontrolled proliferation of cells. These oncogenic lesions deregulate critical intracellular molecular signaling pathways that in turn leads to malignant cell behavior. Advances in our knowledge of the oncogenic lesions that drive cancer have led to the development of small molecules that can disrupt oncogenic signaling pathways. However, the genetic instability of cancer also leads to highly heterogeneous clonal diversity such that a proportion of the cancer cells will contain genetic lesions that will make them resistant to treatment, resulting in transient responses to therapy.

Immunotherapies that exploit the endogenous immune response have been used to treat cancer. Therapies that enhance the ability of immune cells to selectively target cancer cells have been associated with clinical responses in a broad number of tumor types.

Cytotoxic T-Lymphocytes (CTLs) are components of our adaptive immune system that function in a coordinated manner to control and eliminate exogenous threats such as viruses and bacteria in addition to endogenous threats such as cellular transformation events that can lead to cancer. CTLs are a major subpopulation of T-cells that have a killer function and have evolved to recognize and target cells that harbor viruses or potentially harmful traits like cancer. These immune cells are potent and specific. CTLs are able to kill a cell that expresses a low level of an antigen (e.g., between 1-10 antigen molecules) on the cell surface, and are also able to distinguish an antigen amongst millions of potentially similar molecules.

The recognition of tumor cells by the immune system depends on the presence of unique tumor antigens that are recognized by immune cells (e.g., CTLs). A wide variety of cancer antigens have been identified, and are typically classified according to whether they contain sequence changes, are derived from unmutated overexpressed antigens, are derived from developmental antigens, and/or are derived from alterations to posttranslational modifications. Tumor antigenicity is a critical limiting factor that prevents the immune response from successfully targeting cancer. Furthermore, cancers may evolve to limit their antigenicity thereby becoming resistant to immunotherapeutics. Therefore, there is a need for methods to preserve or enhance tumor antigenicity to enable recognition of tumor cells by the immune system.

### Summary of the Invention

We have discovered that phosphoneoantigens are an important class of tumor antigens presented on the surface of some cancer cells, and that these phosphoneoantigens enable selective recognition and targeting of cancer cells by immune cells. By phosphoneoantigen, we refer to any phosphorylated tumor antigen (e.g., a phosphorylated peptide or protein on the surface of a cell) which does not occur in a non-cancerous cell of the same type. Some cancers, however, increase the expression of phosphatases in order to mask these phosphoneoantigens and avoid detection by immune cells. Accordingly, the invention provides methods and compositions for increasing tumor antigenicity by increasing the level of expression of phosphoneoantigens in cells (e.g., restoring the expression of a phosphoneoantigen on the surface of a cancer cell). The invention also provides methods and compositions for enhancing recognition of phosphoneoantigens by immune cells.

In a first aspect, the invention features a method of increasing expression of a phosphoneoantigen in a cancer cell. The method includes the step of contacting a cancer cell with a therapeutic agent that decreases the activity of a cancer-associated phosphatase in the cancer cell. In some embodiments, the cancer cell is a cell in a subject having cancer and contacting the cancer cell with the therapeutic agent is done by administering the therapeutic agent to the subject.

In another aspect, the invention features a method of treating or reducing the probability of occurrence of a cancer in a subject in need thereof, wherein the method includes administering to the subject a therapeutic agent that decreases the activity of a cancer-associated phosphatase in the cells of the cancer.

In some embodiments, administration of the therapeutic agent increases expression of a phosphoneoantigen in the cells of said cancer. In some embodiments, the expression of the phosphoneoantigen is increased by at least 1%, 2%, 5%, 10%, 20%, 30%, 40%, 50%, 60%, 70%, 80%, 90%, 100%, 150%, 200%, 500%, or 1000% relative to a cancer cell of the same type not contacted with the therapeutic agent. In some embodiments, the level of the phosphoneoantigen is restored to the level of a cancer cell not expressing the cancer-associated phosphatase.

In some embodiments, administration of the therapeutic agent decreases the activity of a cancer-associated phosphatase in the cells of the cancer. In some embodiments, the activity of the cancer-associated phosphatase is decreased by at least 1%, 2%, 5%, 10%, 20%, 30%, 40%, 50%, 60%, 70%, 80%, 90%, 100%, 150%, 200%, 500%, or 1000% relative to a cancer cell of the same type not contacted with the therapeutic agent. Activity of the cancer-associated phosphatase may be measured by methods known to one of skill in the art, including by enzymatic activity (e.g., the activity of the cancer-associated phosphatase is decreased by at least 1%, 2%, 5%, 10%, 20%, 30%, 40%, 50%, 60%, 70%, 80%, 90%, 100%, 150%, 200%, 500%, or 1000%), the level of a phosphorylated substrate of the phosphatase (e.g., the level of a phosphorylated substrate, such as a phosphoneoantigen associated with the phosphatase, is increased by at least 1%, 2%, 5%, 10%, 20%, 30%, 40%, 50%, 60%, 70%, 80%, 90%, 100%, 150%, 200%, 500%, or 1000%), or the level of expression of the phosphatase (e.g., the level of expression of the phosphatase is decreased by at least 1%, 2%, 5%, 10%, 20%, 30%, 40%, 50%, 60%, 70%, 80%, 90%, 100%, 150%, 200%, 500%, or 1000%).

In some embodiments, the subject has been previously determined to have increased activity of said cancer-associated phosphatase in the cells of said cancer (e.g., an increased activity level of at least 1%, 2%, 5%, 10%, 20%, 30%, 40%, 50%, 60%, 70%, 80%, 90%, 100%, 150%, 200%, 500%, or 1000%).

In some embodiments, the therapeutic agent is an inhibitor (e.g., a small molecule inhibitor) of the cancer-associated phosphatase. In some embodiments the phosphatase inhibitor is selected from ML095, thiophosphate, L-p-bromotetramisole, tetramisole, levamisole, 5804079, L-phenylalanine, 1-paphthyl phosphate, MLS-0315848, MLS0390945, or MLS-0315687 (CID-715454).

In some embodiments, the method further includes contacting the cancer cell with an immune checkpoint modulator. In some embodiments, wherein the cancer cell is a cell in a subject having cancer, contacting the cancer cell with an immune checkpoint modulator is done by administering the immune checkpoint modulator to the subject. In some embodiments, increasing the expression of the phosphoneoantigen in the cancer cell (e.g., by inhibition of the cancer-associated phosphatase) enhances the immune response against the cancer cell upon administration of the immune checkpoint modulator (e.g. enhances the immune response by 1%, 2%, 5%, 10%, 20%, 30%, 40%, 50%, 60%, 70%, 80%, 90%, 100%, 150%, 200%, 500%, or 1000% or more). In some embodiments, increasing the expression of the phosphoneoantigen in the cancer cell (e.g., by inhibition of the cancer-associated phosphatase) decreases the dose of the immune checkpoint modulator required to achieve a therapeutic effect (e.g., decreases the dose by 1%, 2%, 5%, 10%, 20%, 30%, 40%, 50%, 60%, 70%, 80%, 90%, 100%, 150%, 200%, 500%, or 1000% or more).

In some embodiments, the immune checkpoint modulator is an anti-PD-1 antibody, an anti-CTLA-4 antibody, an anti-TIM-3 antibody, an anti-NKG2A antibody, an anti-LAG-3 antibody, an anti-cMet antibody, an anti-CTLA-4/PD-1 bispecific antibody, an anti-TIM-3/PD-1 bispecific antibody, an anti-PD1/LAG3 bispecific antibody, an anti-PD-1/cMet bispecific antibody, or an antigen-binding fragment thereof.

In some embodiments, the immune checkpoint modulator is pembrolizumab, nivolumab, atelizumab, spartalizumab, camrelizumab, pidilizumab, cemiplimab, tislelizumab, JS001, MEDI0680, BCD-100, JNJ-63723283, CBT-501, TSR-042, MGA012, PF-06801591, KN035, LZM009, XmAb20717, AGEN2034, Sym021, AK105, AK104, HLX10, CS1003, STI-1110, MGD013, MCLA-134, AM001 , or EDI5752.

In some embodiments, the method further includes contacting the cancer cell with an immunomodulatory cytokine. The immunomodulatory cytokine may be selected from an interferon (e.g., IFNα, IFNβ, or IFNγ), an interleukin (e.g., IL-2, IL-12, or IL-15), or a synthetic mimic of a naturally-occurring cytokine (e.g., Neo-2/15, which mimics IL-2 and IL-15 activity). In some embodiments, wherein the cancer cell is a cell in a subject having cancer, contacting the cancer cell with an immunomodulatory cytokine is done by administering the immunomodulatory cytokine to the subject. In some embodiments, increasing the expression of the phosphoneoantigen in the cancer cell (e.g., by inhibition of the cancer-associated phosphatase) enhances the immune response against the cancer cell upon administration of the immunomodulatory cytokine (e.g. enhances the immune response by 1%, 2%, 5%, 10%, 20%, 30%, 40%, 50%, 60%, 70%, 80%, 90%, 100%, 150%, 200%, 500%, or 1000% or more). In some embodiments, increasing the expression of the phosphoneoantigen in the cancer cell (e.g., by inhibition of the cancer-associated phosphatase) decreases the dose of the immunomodulatory cytokine required to achieve a therapeutic effect (e.g., decreases the dose by 1%, 2%, 5%, 10%, 20%, 30%, 40%, 50%, 60%, 70%, 80%, 90%, 100%, 150%, 200%, 500%, or 1000% or more).

In some embodiments, the method further includes contacting the cancer cell with an agent that inhibits myeloid derived suppressor cells. The agent that inhibits myeloid derived suppressor cells (MDSCs) may be selected from any agent that inhibits myeloid derived suppressor cells known to those of skill in the art, e.g., an indoleamine 2,3-dioxygenase (IDO) inhibitor, an anti-VEGF antibody, a nitric oxide synthetase (NOS) inhibitor, a CSF-1R inhibitor, an arginase inhibitor, or a multi-kinase inhibitor. In some embodiments, wherein the cancer cell is a cell in a subject having cancer, contacting the cancer cell with an agent that inhibits myeloid derived suppressor cells is done by administering the agent that inhibits myeloid derived suppressor cells to the subject. In some embodiments, increasing the expression of the phosphoneoantigen in the cancer cell (e.g., by inhibition of the cancer-associated phosphatase) enhances the immune response against the cancer cell upon administration of the agent that inhibits myeloid derived suppressor cells (e.g. enhances the immune response by 1%, 2%, 5%, 10%, 20%, 30%, 40%, 50%, 60%, 70%, 80%, 90%, 100%, 150%, 200%, 500%, or 1000% or more). In some embodiments, increasing the expression of the phosphoneoantigen in the cancer cell (e.g., by inhibition of the cancer-associated phosphatase) decreases the dose of the agent that inhibits myeloid derived suppressor cells required to achieve a therapeutic effect (e.g., decreases the dose by 1%, 2%, 5%, 10%, 20%, 30%, 40%, 50%, 60%, 70%, 80%, 90%, 100%, 150%, 200%, 500%, or 1000% or more).

In some embodiments, the method further includes vaccinating the subject with the phosphoneoantigen and, optionally, a suitable adjuvant (e.g., administering to the subject a phosphoneoantigen and, optionally, a suitable adjuvant, in an amount sufficient to induce and immune response against the phosphoneoantigen). In some embodiments, increasing the expression of the phosphoneoantigen in the cancer cell (e.g., by inhibition of the cancer-associated phosphatase) enhances the immune response against the cancer cell upon vaccination with the phosphoneoantigen (e.g. enhances the immune response by 1%, 2%, 5%, 10%, 20%, 30%, 40%, 50%, 60%, 70%, 80%, 90%, 100%, 150%, 200%, 500%, or 1000% or more). In some embodiments, increasing the expression of the phosphoneoantigen in the cancer cell (e.g., by inhibition of the cancer-associated phosphatase) decreases the dose of the vaccination with the phosphoneoantigen required to achieve a therapeutic effect (e.g., decreases the dose by 1%, 2%, 5%, 10%, 20%, 30%, 40%, 50%, 60%, 70%, 80%, 90%, 100%, 150%, 200%, 500%, or 1000% or more).

In some embodiments, the method further includes administering to said subject a T-cell that has been genetically modified to express a T-cell receptor (TCR), wherein said TCR binds specifically to the phosphoneoantigen. In some embodiments, increasing the expression of the phosphoneoantigen in the cancer cell (e.g., by inhibition of the cancer-associated phosphatase) enhances the immune response against the cancer cell upon administration of the genetically engineered T-cell (e.g. enhances the immune response by 1%, 2%, 5%, 10%, 20%, 30%, 40%, 50%, 60%, 70%, 80%, 90%, 100%, 150%, 200%, 500%, or 1000% or more). In some embodiments, increasing the expression of the phosphoneoantigen in the cancer cell (e.g., by inhibition of the cancer-associated phosphatase) decreases the dose of the genetically engineered T-cell required to achieve a therapeutic effect (e.g., decreases the dose by 1%, 2%, 5%, 10%, 20%, 30%, 40%, 50%, 60%, 70%, 80%, 90%, 100%, 150%, 200%, 500%, or 1000% or more).

In some embodiments, the method further includes contacting the cancer cell with an anti-proliferative agent. In some embodiments, wherein the cancer cell is a cell in a subject having cancer, contacting the cancer cell with an anti-proliferative agent is done by administering the anti-proliferative agent to the subject.

In some embodiments, the anti-proliferative agent is MK-2206, ON 013105, RTA 402, BI 2536, Sorafenib, ISIS-STAT3Rx, a microtubule inhibitor, a topoisomerase inhibitor, a platin, an alkylating agent, an anti-metabolite, paclitaxel, gemcitabine, doxorubicin, vinblastine, etoposide, 5-fluorouracil, carboplatin, altretamine, aminoglutethimide, amsacrine, anastrozole, azacitidine, bleomycin, busulfan, carmustine, chlorambucil, 2-chlorodeoxyadenosine, cisplatin, colchicine, cyclophosphamide, cytarabine, cytoxan, dacarbazine, dactinomycin, daunorubicin, docetaxel, estramustine phosphate, floxuridine, fludarabine, gentuzumab, hexamethylmelamine, hydroxyurea, ifosfamide, imatinib, interferon, irinotecan, lomustine, mechlorethamine, melphalan, 6-mercaptopurine, methotrexate, mitomycin, mitotane, mitoxantrone, pentostatin, procarbazine, rituximab, streptozocin, tamoxifen, temozolomide, teniposide, 6-thioguanine, topotecan, trastuzumab, vincristine, vindesine, or vinorelbine.

In some embodiments, the cancer cell has been previously determined to have increased activity of the cancer-associated phosphatase (e.g., 1%, 2%, 5%, 10%, 20%, 30%, 40%, 50%, 60%, 70%, 80%, 90%, 100%, 150%, 200%, 500%, or 1000% or greater activity relative to a wild-type cell of the same type). Activity of the cancer-associated phosphatase may be measured by methods known to one of skill in the art, including by enzymatic activity(e.g., the activity of the cancer-associated phosphatase is decreased by at least 1%, 2%, 5%, 10%, 20%, 30%, 40%, 50%, 60%, 70%, 80%, 90%, 100%, 150%, 200%, 500%, or 1000%), the level of a phosphorylated substrate of the phosphatase (e.g., the level of a phosphorylated substrate, such as a phosphoneoantigen associated with the phosphatase, is increased by at least 1%, 2%, 5%, 10%, 20%, 30%, 40%, 50%, 60%, 70%, 80%, 90%, 100%, 150%, 200%, 500%, or 1000%), or the level of expression of the phosphatase (e.g., the level of expression of the phosphatase is decreased by at least 1%, 2%, 5%, 10%, 20%, 30%, 40%, 50%, 60%, 70%, 80%, 90%, 100%, 150%, 200%, 500%, or 1000%).

In another aspect, the invention features a method of identifying a subject at risk of developing cancer, wherein the method includes determining the activity of a cancer-associated phosphatase in a cell of said subject, wherein increased activity of said cancer-associated phosphatase (e.g., an increase of 1%, 2%, 5%, 10%, 20%, 30%, 40%, 50%, 60%, 70%, 80%, 90%, 100% or more relative to a wild-type cell of the same type) indicates an increased risk of developing cancer.

In some embodiments, if said subject is determined to have an increased risk of developing cancer, the subject is administered a therapeutic agent that decreases the activity of the cancer-associated phosphatase in the cells of the cancer.

In some embodiments, the therapeutic agent is an inhibitor (e.g., a small molecule inhibitor) of the cancer-associated phosphatase. In some embodiments the phosphatase inhibitor is selected from ML095, thiophosphate, L-p-bromotetramisole, tetramisole, levamisole, 5804079, L-phenylalanine, 1-paphthyl phosphate, MLS-0315848, MLS0390945, or MLS-0315687 (CID-715454).

In some embodiments, if said subject is determined to have an increased risk of developing cancer, said subject is vaccinated with a phosphoneoantigen and a suitable adjuvant.

In another aspect, the invention features a method of producing a nucleic acid encoding a TCR that binds to a phosphoneoantigen. The method, in general, includes the steps of:
(a) immunizing a mammal, except for a human, with a phosphoneoantigen, wherein the immunizing optionally further comprises administering an adjuvant;
(b) isolating a cell from the mammal of step (a) that expresses a TCR that binds to the phosphoneoantigen antigen; and
(c) isolating a nucleic acid from the cell of step (b) that encodes the TCR that binds to the phosphoneoantigen antigen.

In some embodiments, the method further includes expressing the nucleic acid encoding the TCR that binds to the phosphoneoantigen in a host cell, thereby producing a TCR that binds to said phosphoneoantigen.

In another aspect, the invention features a pharmaceutical composition comprising a T-cell, wherein the T-cell has been genetically modified to express a TCR, wherein the TCR binds specifically to a phosphoneoantigen.

In another aspect, the invention features a method of treating a cancer in a subject in need thereof, wherein said method includes administering to said subject a T-cell that has been genetically modified to express a TCR, wherein the TCR binds specifically to a phosphoneoantigen.

In another aspect, the invention features a method of treating or reducing the probability of occurrence of a cancer in a subject in need thereof, wherein the method includes vaccinating the subject with a phosphoneoantigen and, optionally, a suitable adjuvant.

In some embodiments of any of the foregoing aspects, the cancer-associated phosphatase is an alkaline phosphatase. In some embodiments, the alkaline phosphatase is selected from placental alkaline phosphatase (ALPP), placental-like alkaline phosphatase (ALPPL2), intestinal alkaline phosphatase (ALPI), or tissue-nonspecific alkaline phosphatase (ALPL).

In some embodiments of any of the foregoing aspects, the cancer-associated phosphatase is an acid phosphatase. In some embodiments, the acid phosphatase is prostatic acid phosphatase (ACPP).

In some embodiments of any of the foregoing aspects, the cancer-associated phosphatase is a protein serine/threonine phosphatase (PSP).

In some embodiments of any of the foregoing aspects, an increase in the phosphoneoantigen is associated with increased activity of the cancer-associated phosphatase (e.g., increased activity or increase expression of the cancer-associated phosphatase).

In some embodiments of any of the foregoing aspects, the cancer is selected from acute leukemia, acute lymphocytic leukemia, acute myelocytic leukemia, acute myeloblastic leukemia, acute promyelocytic leukemia, acute myelomonocytic leukemia, acute monocytic leukemia, acute erythroleukemia, chronic leukemia, chronic myelocytic leukemia, chronic lymphocytic leukemia, Hodgkin's disease, non-Hodgkin's disease, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, uterine cancer, testicular cancer, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodenroglioma, schwannoma, meningioma, melanoma, neuroblastoma, or retinoblastoma.

### Brief Description of the Figures

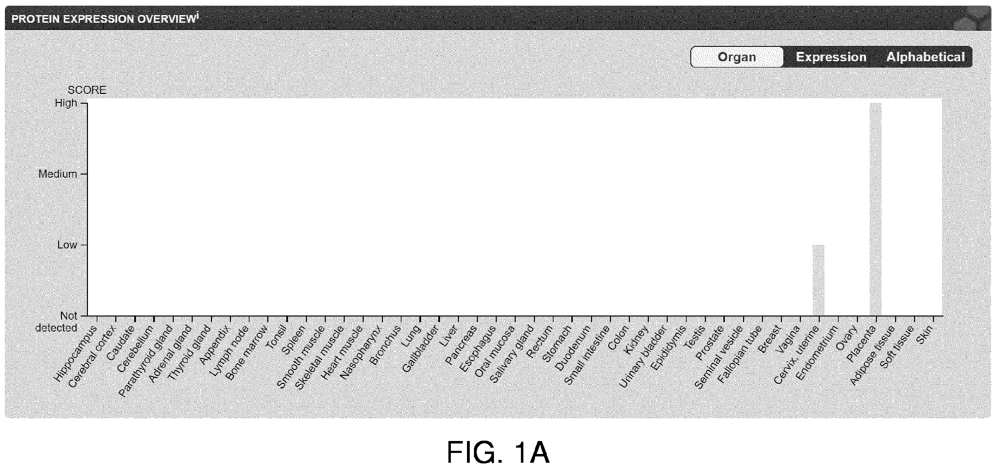

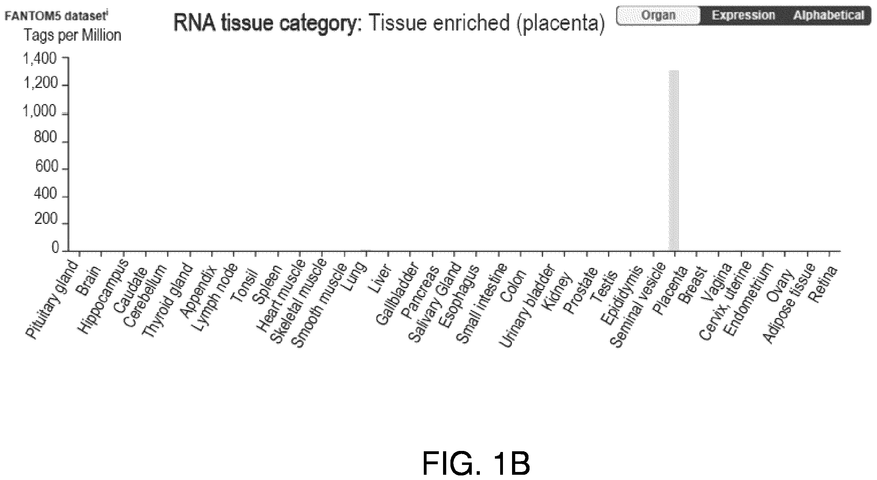

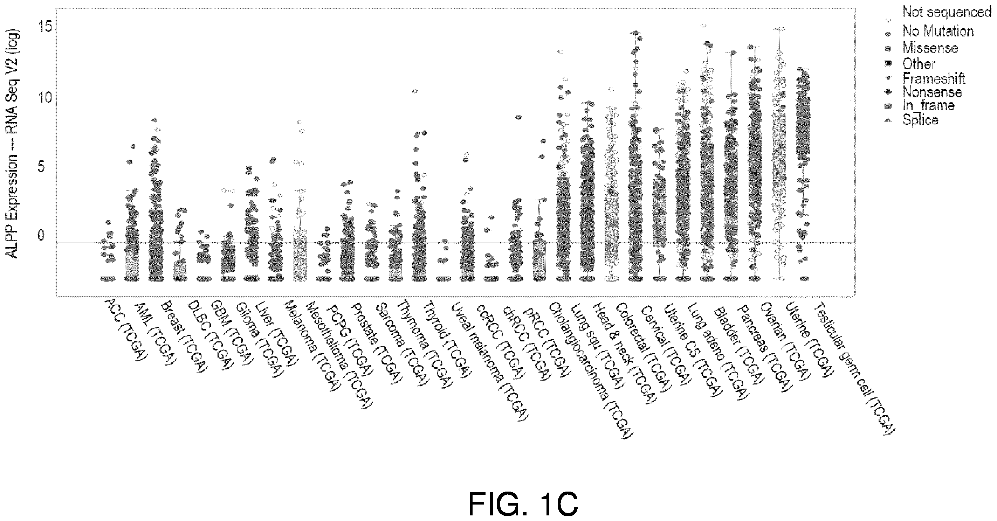

**Figs. 1A-C** are series of images showing the expression of ALPP in healthy and cancerous tissues. Fig. 1A shows protein expression as defined from the Human Protein Atlas in 35 healthy tissues (http://www.proteinatals.org/). Fig. 1B shows RNA expression in the FANTOM5 series of healthy tissue samples. Fig. 1C shows RNA expression of ALPP in 30 cancer types taken from the cBioPortal for Cancer Genomics (http://www.cbioportal.org).

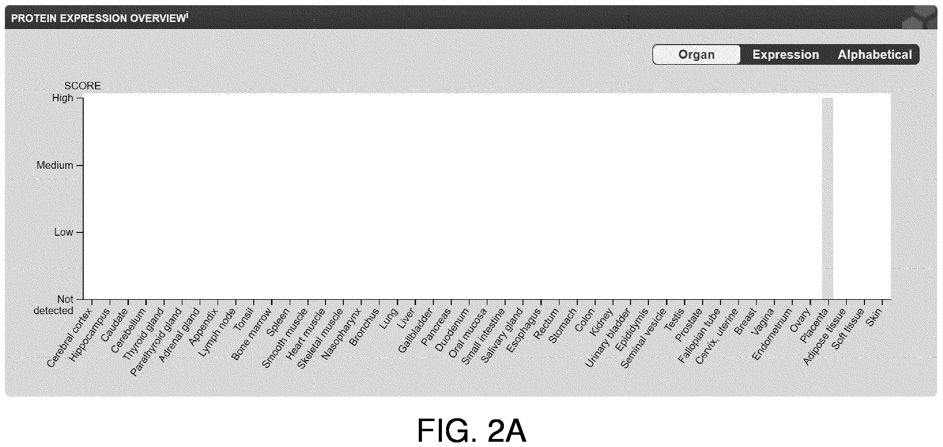

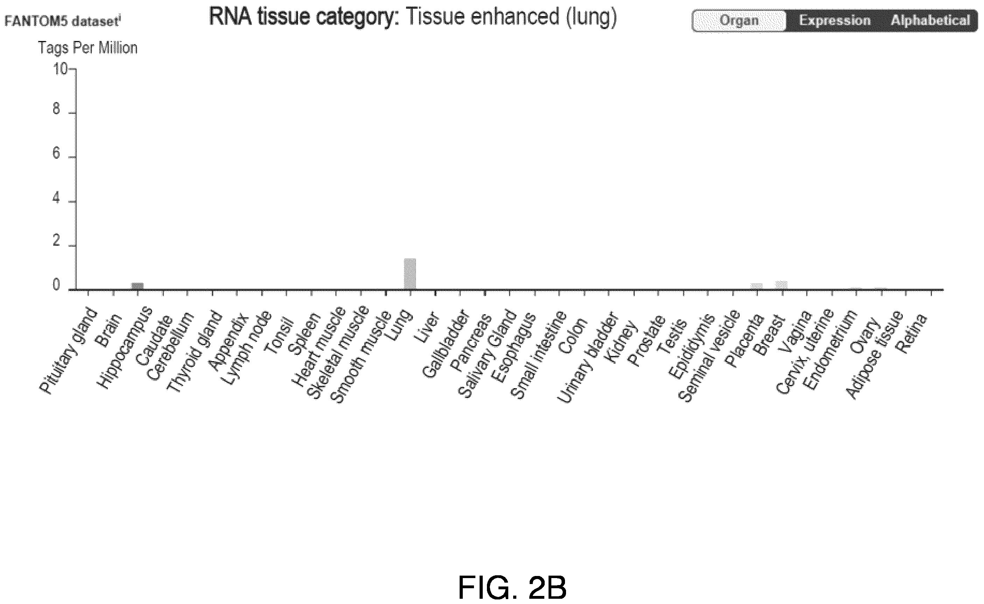

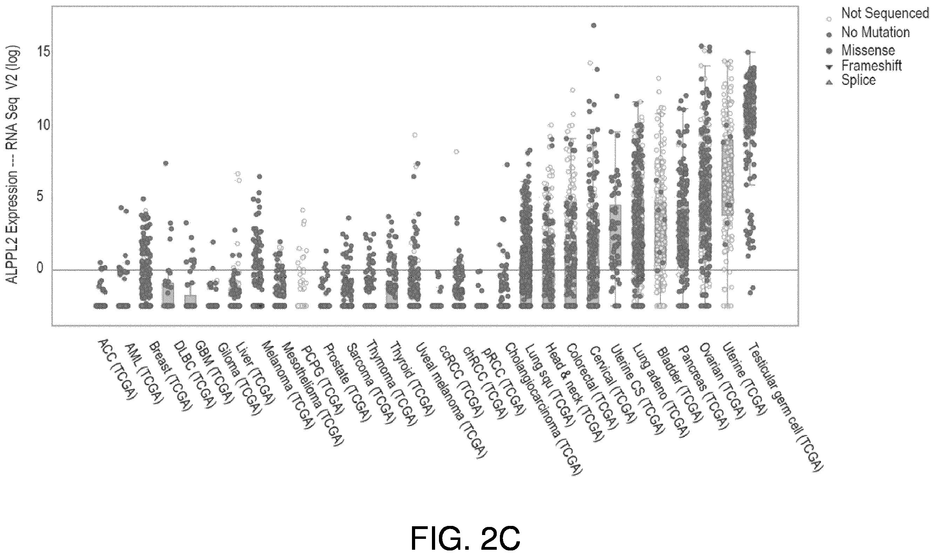

**Figs. 2A-C** are a series of images showing the expression of ALPPL2 in healthy and cancerous tissues. Fig. 2A shows protein expression as defined from the Human Protein Atlas in 35 healthy tissues (http://www.proteinatals.org/). Fig. 2B shows RNA expression in the FANTOM5 series of healthy tissue samples. Fig. 2C shows RNA expression of ALPPL2 in 30 cancer types taken from the cBioPortal for Cancer Genomics (http://www.cbioportal.org).

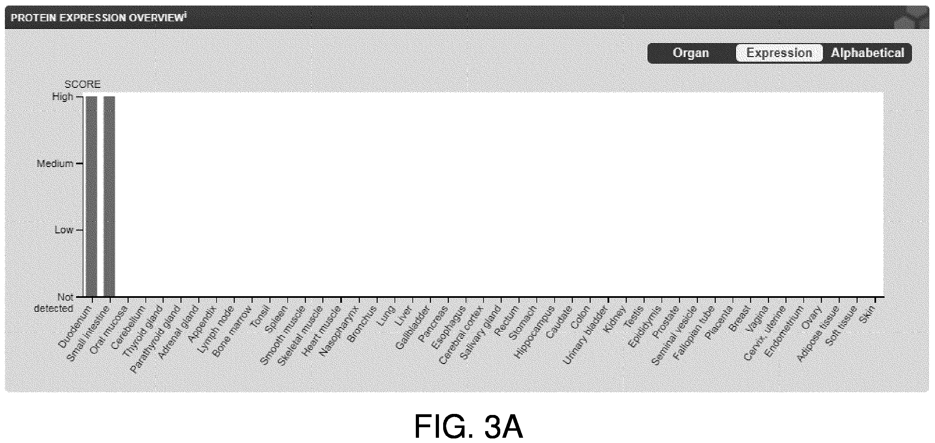

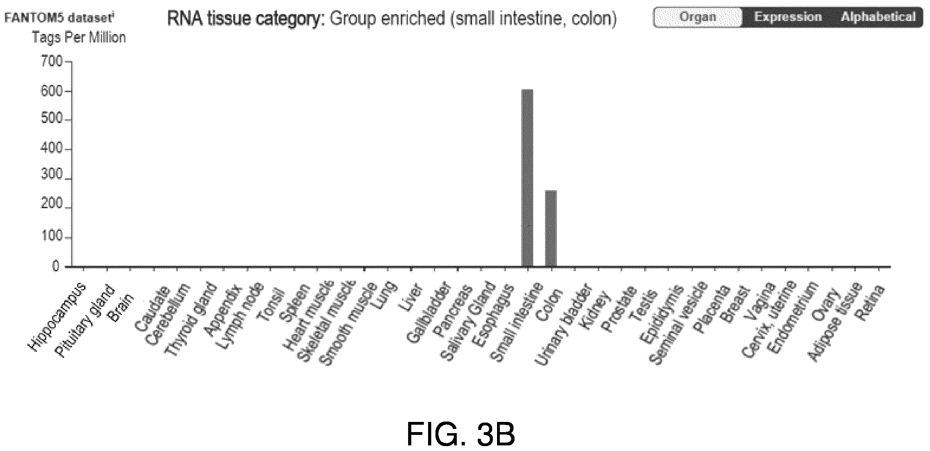

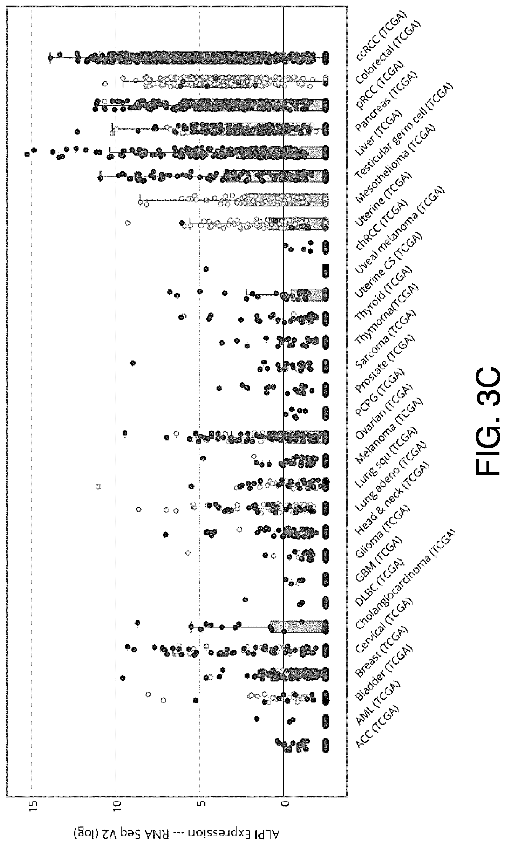

**Figs. 3A-C** are a series of images showing the expression of ALPI in healthy and cancerous tissues. Fig. 3A shows protein expression as defined from the Human Protein Atlas in 35 healthy tissues (http://www.proteinatlas.org/). Fig. 3B shows RNA expression in the FANTOM5 series of healthy tissue samples. Fig. 3C shows RNA expression of ALPI in 30 cancer types taken from the cBioPortal for Cancer Genomics (http://www.cbioportal.org).

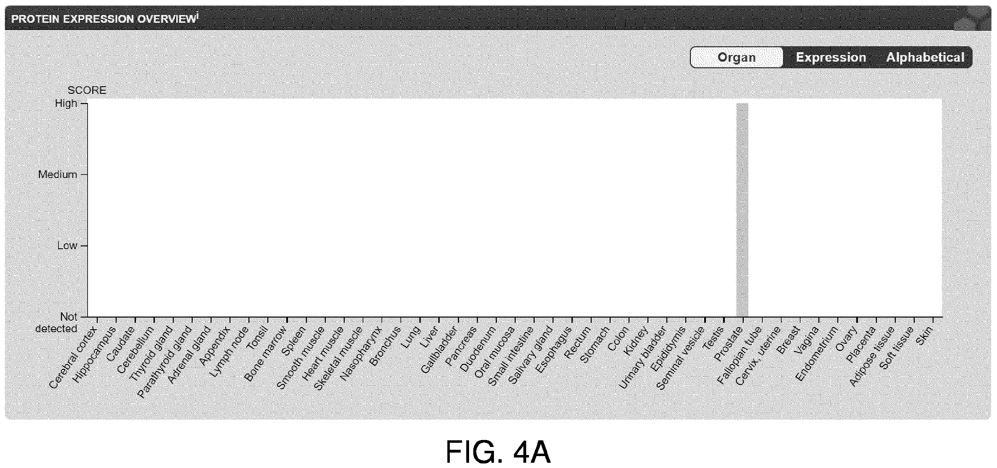

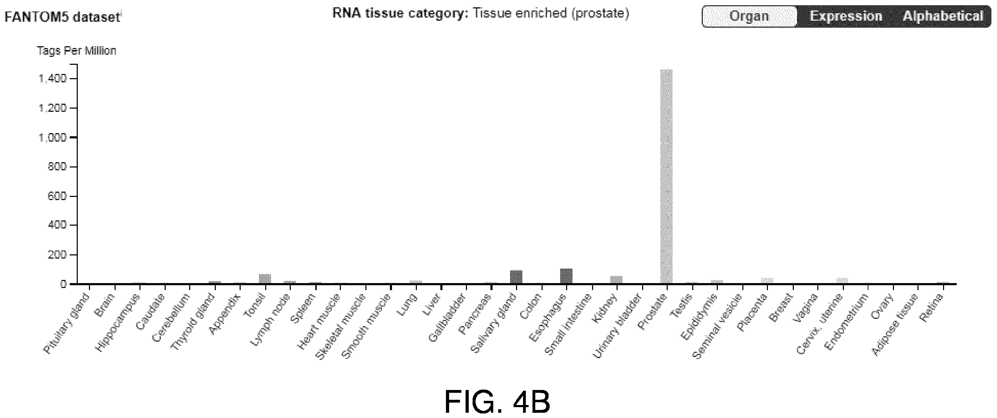

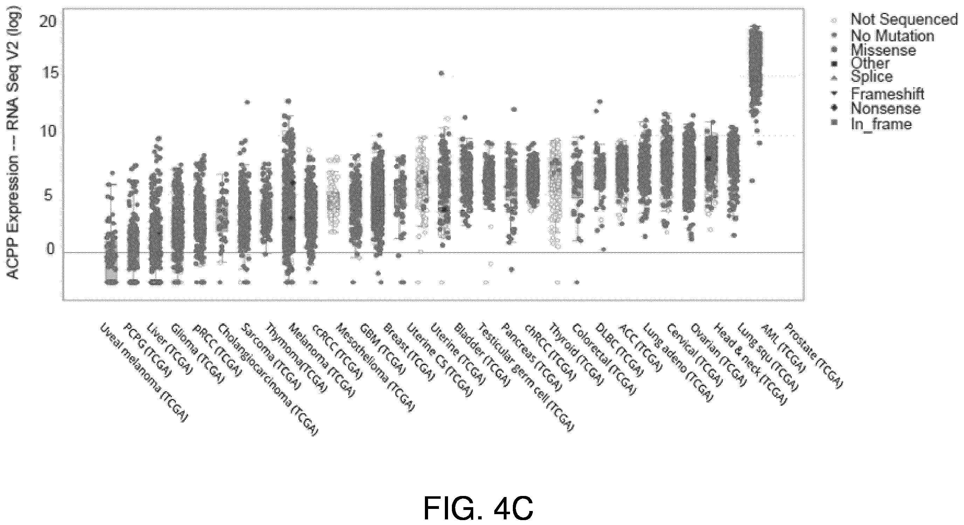

**Figs. 4A-C** are a series of images showing the expression of ACPP in healthy and cancerous tissues. Fig. 4A shows protein expression as defined from the Human Protein Atlas in 35 healthy tissues (http://www.proteinatlas.org/). Fig. 4B shows RNA expression in the FANTOM5 series of healthy tissue samples. Fig. 4C shows RNA expression of ACPP in 30 cancer types taken from the cBioPortal for Cancer Genomics (http://www.cbioportal.org) demonstrating high prostate expression.

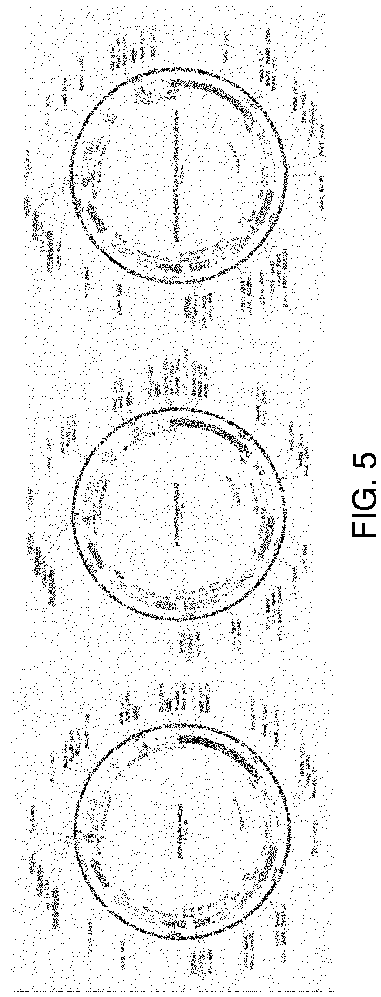

**Fig. 5** is a series of images depicting lentivector constructs for ALPP, ALPPL2 and luciferase expression. ALPP (left) and Luciferase (right) vectors are tagged with GFP; ALPPL2 vector (center) is tagged with mCherry.

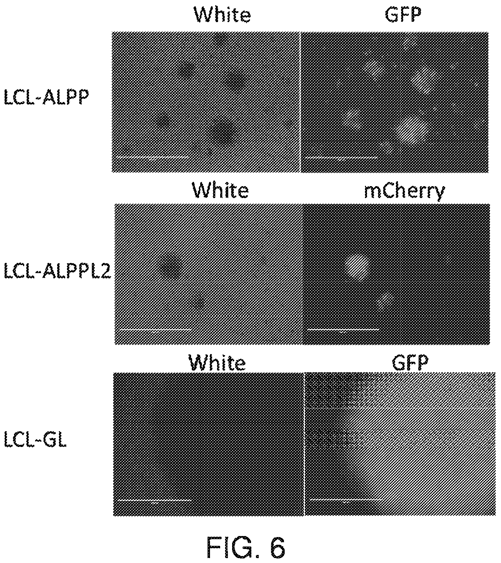

**Fig. 6** is a series of images showing the labeling of a lymphoblastoid cell line (LCL) with ALPP, ALPPL2, or luciferase. Three weeks post transduction, purified LCL-ALPP, LCL-ALPPL2, or LCL-GL cells were imaged by microscopy. Top panel: LCL-ALPP line was consistently showing green (GFP); middle panel: LCL-ALPPL2 line was consistently showing red (mCherry); bottom panel: LCL-GL line was consistently showing green (GFP).

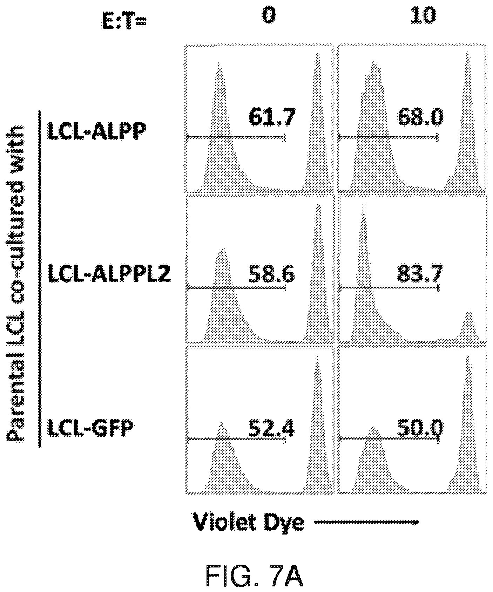

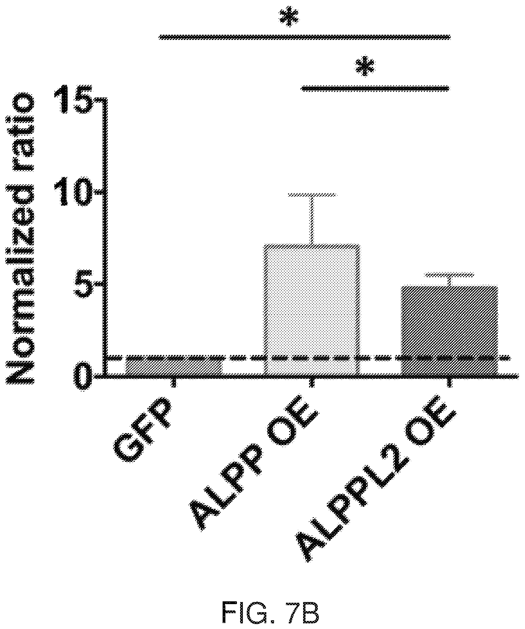

**Figs. 7A-B** show that ALPP or ALPPL2 expression inhibits specific CD8⁺ T-cell responses against autologous LCL cells. Violet dye pre-labeled LCL cells were co-cultured with either LCL-ALPP, LCL-ALPPL2 or LCL-GL (at ratio 1:1) in the presence (10:1) or absence (0:1) of autologous specific CD8⁺ T-cells. Sixteen hours later, the cells were harvested and FACS was used to determine the ratios between parent LCL and either LCL-ALPP, LCL-ALPPL2 or LCL-GL. Fig. 7A is a series of flow cytometry traces showing the ratios between LCL-ALPP, LCL-ALPPL2, or LCL-GL to parent LCL. Fig. 7B is a graph corresponding to the flow cytometry data of Fig. 7A.

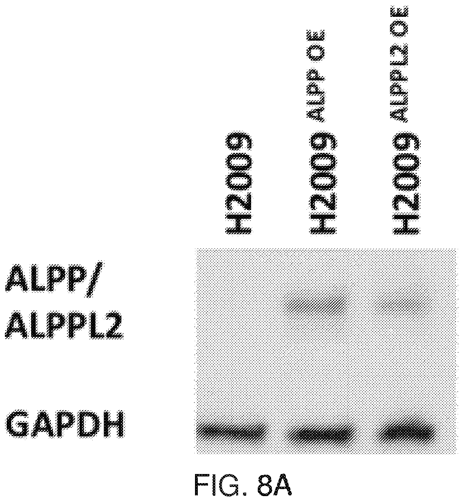

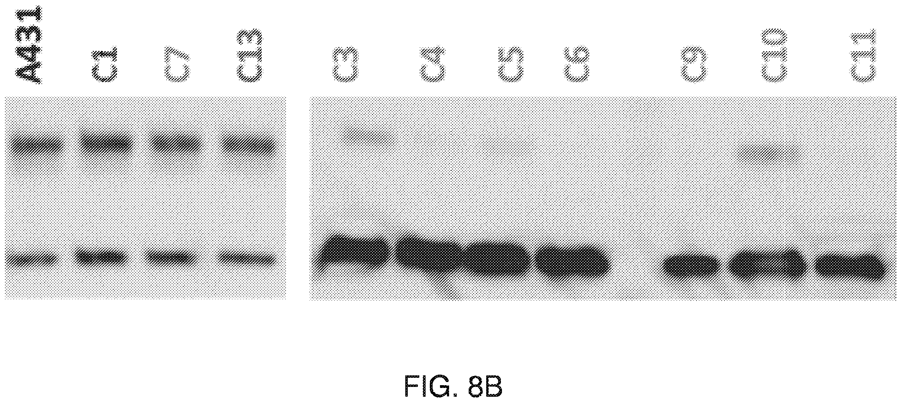

**Figs. 8A-B** are a series of western-blots showing the verification of ALPP or ALPPL2 expressions in lentirviral engineered H2009 and A431 haploidentical cell lines. ALPP and ALPPL2 expressions were verified by western-blot in H2009 (Fig. 8A) and A431 (Fig. 8B) cell lines. For A431: C1 and C3-C6 are single clones for A431^{ALPP KO}; C7 and C9-C11 are single clones for A431^{ALPPL2 KO}; C13 is a single clone for scramble knock-out.

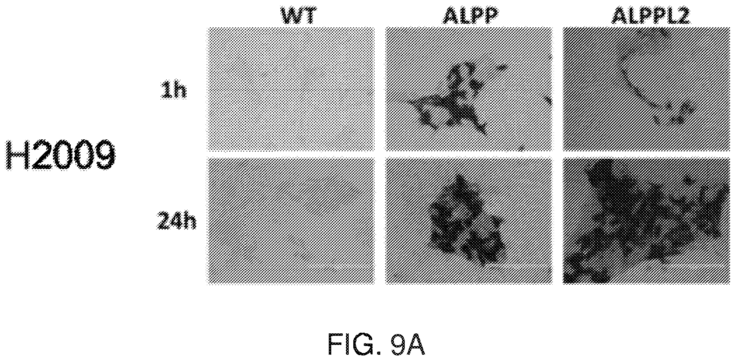

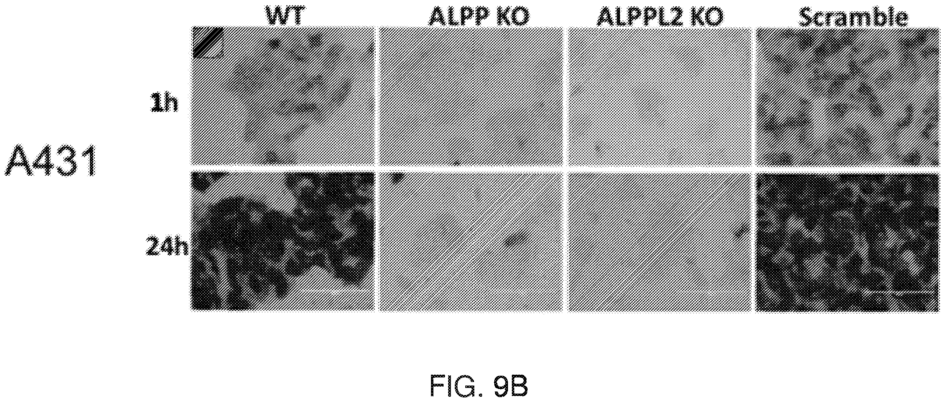

**Figs. 9A-B** are a series if images showing the use of an NBT-BCIP reaction assay to verify ALPP or ALPPL2 expressions in lentiviral engineered H2009 (Fig. 9A) and A431 (Fig. 9B) haploidentical cell lines. Cells positive for either ALPP or ALPPL2 are stained with dark brown color. Here, clone C6 was used for A431 ^{ALPP KO}; clone C9 was used for A431 ^{ALPPL2 KO}; and clone C13 was used for A431 ^{scramble}.

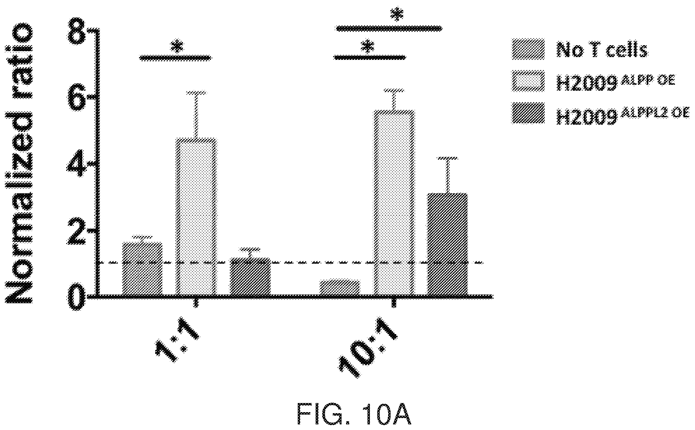

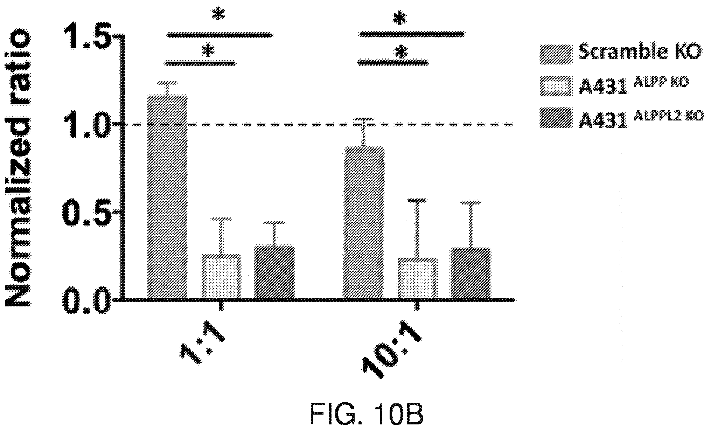

**Figs. 10A-B** are a series of graphs showing that ALPP or ALPPL2 expression inhibits specific T-cell killing of HLA-matched haploidentical targets. Violet dye pre-labeled parental tumor cell lines were seeded together with lentiviral engineered cell lines (at 1:1 ratio) in the presence (CD8:LCLs=1 :1, 10:1) or absence (CD8:LCLs=0:1) of autologous specific CD8 T cells in 96 well plates. After sixteen hours, cells were tested by FACS for calculating the ratios between lentiviral engineered cell line and parental line. Statistical results for H2009 (Fig. 10A) and A431 (Fig. 10B) are shown. A normalized ratio (y-axis) equal to 1 indicates no preferential killing for either tumor cell lines; a normalized ration of greater than 1 indicates more killing of parental lines; a normalized ratio of less than 1 indicates less killing of parental lines. All results were normalized to conditions without the presence of specific T-.cells. * indicates *p*<0.05.

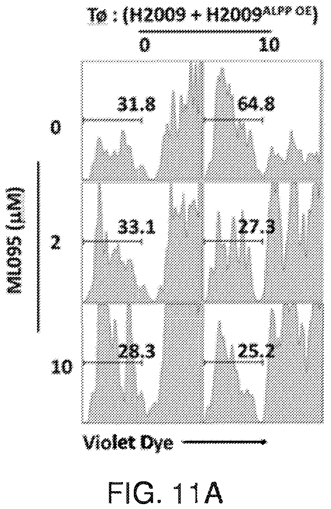

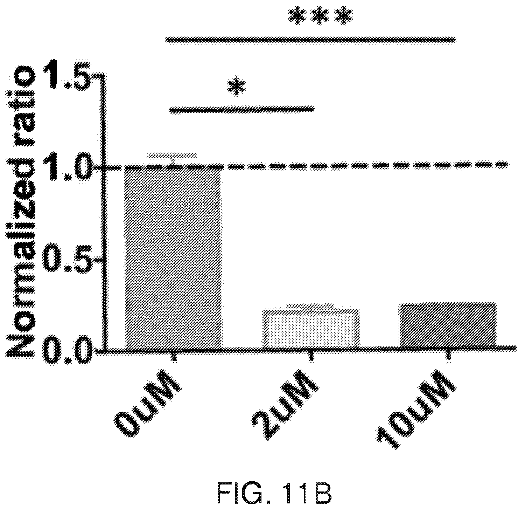

**Figs. 11A-B** show that ML095 recovers specific CD8⁺ T-cell killing of H2009^{ALPP OE}. Fig. 11A is a series of FACS traces. Fig. 11B is a graph corresponding to Fig. 11A.

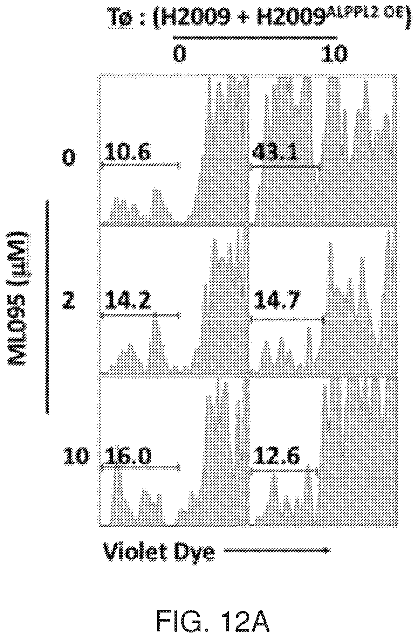

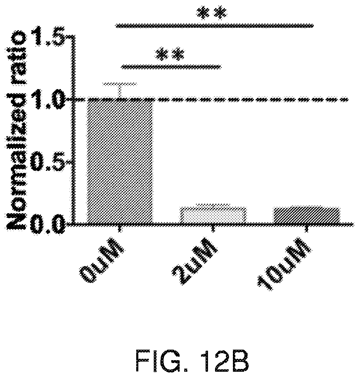

**Figs. 12A-B** show that ML095 recovers specific CD8⁺ T-cell killing of H2009^{ALPP2 OE}. Fig. 12A is a series of FACS traces. Fig. 12B is a graph corresponding to Fig. 12A.

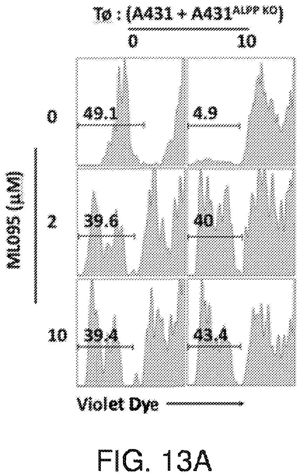

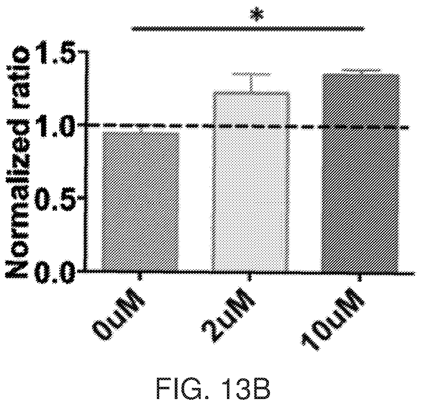

**Figs. 13A-B** show that ML095 increases specific CD8⁺ T-cell killing of A431 as compared to A431^{ALPP KO}. Fig. 13A is a series of FACS traces. Fig. 13B is a graph corresponding to Fig. 13A.

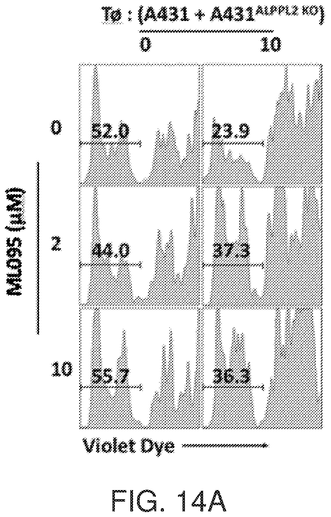

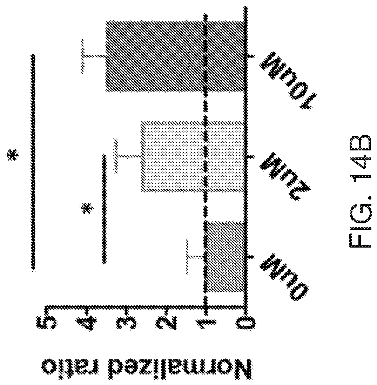

**Figs. 14A-B** show that ML095 increases specific CD8⁺ T-cell killing of A431 as compared to A431^{ALPP2 KO}. Fig. 14A is a series of FACS traces. Fig. 14B is a graph corresponding to Fig. 14A.

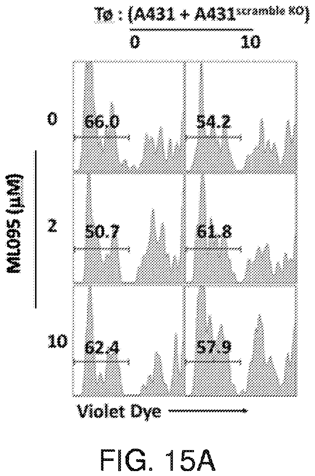

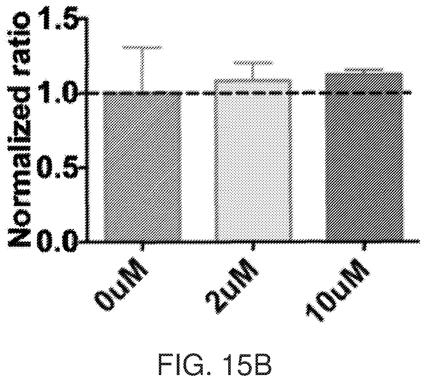

**Figs. 15A-B** show that ML095 does not impact specific CD8⁺ T-cell killing of A431 as compared to A431^{scramble KO}. Fig. 15A is a series of FACS traces. Fig. 15B is a graph corresponding to Fig. 15A.

### Definitions

To facilitate the understanding of this invention, a number of terms are defined below. Terms defined herein have meanings as commonly understood by a person of ordinary skill in the areas relevant to the present invention. Terms such as "a", "an," and "the" are not intended to refer to only a singular entity, but include the general class of which a specific example may be used for illustration. The terminology herein is used to describe specific embodiments of the invention, but their usage does not delimit the invention, except as outlined in the claims.

As used herein, the term "about" refers to a value that is within 10% above or below the value being described.

As used here, any values provided in a range of values include both the upper and lower bounds, and any values contained within the upper and lower bounds.

As used herein, "administration" refers to providing or giving a subject a therapeutic agent (e.g., a phosphatase inhibitor, a phosphoneoantigen, or any therapeutic agent described herein), by any effective route. Exemplary routes of administration are described herein below.

As used herein, the term "inhibitor" refers to an agent (e.g., a small molecule or antibody) that reduces or inhibits enzymatic activity. An inhibitor may reduce enzymatic activity by directly binding to the enzyme, by blocking the enzyme binding site, by modulating enzymatic conformation. An inhibitor may reduce enzymatic activity by 1%, 2%, 5%, 10%, 20%, 30%, 40%, 50%, 60%, 70%, 80%, 90%, 95%, 98%,100% or more. An inhibitor may also completely block or inhibit enzymatic activity. Inhibitor activity may be concentration-dependent or -independent.

As used herein, the term "antibody" refers to a molecule that specifically binds to, or is immunologically reactive with, a particular antigen and includes at least the variable domain of a heavy chain, and normally includes at least the variable domains of a heavy chain and of a light chain of an immunoglobulin. Antibodies and antigen-binding fragments, variants, or derivatives thereof include, but are not limited to, polyclonal, monoclonal, multispecific, human, humanized, primatized, or chimeric antibodies, heteroconjugate antibodies (e.g., bi- tri- and quad-specific antibodies, diabodies, triabodies, and tetrabodies), single-domain antibodies (sdAb), epitope-binding fragments, e.g., Fab, Fab' and F(ab')₂, Fd, Fvs, single-chain Fvs (scFv), rlgG, single-chain antibodies, disulfide-linked Fvs (sdFv), fragments containing either a V_{L} or V_{H} domain, fragments produced by an Fab expression library, and anti-idiotypic (anti-ld) antibodies. Antibody molecules described herein can be of any type (e.g., IgG, IgE, IgM, IgD, IgA, and IgY), class (e.g., IgG1, IgG2, IgG3, IgG4, IgA1 and IgA2), or subclass of immunoglobulin molecule. Moreover, unless otherwise indicated, the term "monoclonal antibody" (mAb) is meant to include both intact molecules as well as antibody fragments (such as, for example, Fab and F(ab')₂ fragments) that are capable of specifically binding to a target protein. Fab and F(ab')₂ fragments lack the Fc fragment of an intact antibody.

The term "antigen-binding fragment," as used herein, refers to one or more fragments of an immunoglobulin that retain the ability to specifically bind to a target antigen. The antigen-binding function of an immunoglobulin can be performed by fragments of a full-length antibody. The antibody fragments can be a Fab, F(ab')₂, scFv, SMIP, diabody, a triabody, an affibody, a nanobody, an aptamer, or a domain antibody. Examples of binding fragments encompassed by the term "antigen-binding fragment" of an antibody include, but are not limited to: (i) a Fab fragment, a monovalent fragment consisting of the V_{L}, V_{H}, C_{L}, and C_{H}1 domains; (ii) a F(ab')₂ fragment, a bivalent fragment containing two Fab fragments linked by a disulfide bridge at the hinge region; (iii) a Fd fragment consisting of the V_{H} and C_{H}1 domains; (iv) a Fv fragment consisting of the V_{L} and V_{H} domains of a single arm of an antibody, (v) a sdAb (Ward et al., Nature 341:544-546, 1989) including V_{H} and V_{L} domains; (vi) a sdAb fragment that consists of a V_{H} domain; (vii) a sdAb that consists of a V_{H} or a V_{L} domain; (viii) an isolated complementarity determining region (CDR); and (ix) a combination of two or more isolated CDRs which may optionally be joined by a synthetic linker. Furthermore, although the two domains of the Fv fragment, V_{L} and V_{H}, are coded for by separate genes, they can be joined, using recombinant methods, by a linker that enables them to be made as a single protein chain in which the V_{L} and V_{H} regions pair to form monovalent molecules (known as single chain Fv (scFv)). These antibody fragments can be obtained using conventional techniques known to those of skill in the art, and the fragments can be screened for utility in the same manner as intact antibodies. Antigen-binding fragments can be produced by recombinant DNA techniques, enzymatic or chemical cleavage of intact immunoglobulins, or, in certain cases, by chemical peptide synthesis procedures known in the art.

As used herein, the term "cell type" refers to a group of cells sharing a phenotype that is statistically separable based on gene expression data. For instance, cells of a common cell type may share similar structural and/or functional characteristics, such as similar gene activation patterns and antigen presentation profiles. Cells of a common cell type may include those that are isolated from a common tissue (e.g., epithelial tissue, neural tissue, connective tissue, or muscle tissue) and/or those that are isolated from a common organ, tissue system, blood vessel, or other structure and/or region in an organism.

As used herein, a "combination therapy" or "administered in combination" means that two or more different agents or treatments are administered to a subject as part of a defined treatment regimen for a particular disease or condition. The treatment regimen defines the doses and periodicity of administration of each agent such that the effects of the separate agents on the subject overlap. In some embodiments, the delivery of the two or more agents is simultaneous or concurrent and the agents may be co-formulated. In other embodiments, the two or more agents are not co-formulated and are administered in a sequential manner as part of a prescribed regimen. In some embodiments, administration of two or more agents or treatments in combination is such that the reduction in a symptom, or other parameter related to the disorder is greater than what would be observed with one agent or treatment delivered alone or in the absence of the other. The effect of the two treatments can be partially additive, wholly additive, or greater than additive (e.g., synergistic). Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, a first therapeutic agent of the combination may be administered by intravenous injection while a second therapeutic agent of the combination may be administered orally.

As used herein, the terms "effective amount," "therapeutically effective amount," and a "sufficient amount" of a composition, small molecule, peptide, vaccine, antibody, vector construct, viral vector, cell, or any therapeutic agent described herein refer to a quantity sufficient to, when administered to a subject, including a mammal (e.g., a human), effect beneficial or desired results, including effects at the cellular level, tissue level, or clinical results, and, as such, an "effective amount" or synonym thereto depends upon the context in which it is being applied. For example, in the context of treating cancer it is an amount of the composition, small molecule, peptide, vaccine, antibody, vector construct, viral vector, cell, or therapeutic agent sufficient to achieve a treatment response as compared to the response obtained without administration of the composition, small molecule, peptide, vaccine, antibody, vector construct, viral vector, cell, or therapeutic agent. The amount of a given composition described herein that will correspond to such an amount will vary depending upon various factors, such as the given agent, the pharmaceutical formulation, the route of administration, the type of disease or disorder, the identity of the subject (e.g., age, sex, weight) or host being treated, and the like, but can nevertheless be routinely determined by one skilled in the art. Also, as used herein, a "therapeutically effective amount" of a composition, small molecule, peptide, vaccine, antibody, vector construct, viral vector, cell, or therapeutic agent of the present disclosure is an amount that results in a beneficial or desired result in a subject as compared to a control. As defined herein, a therapeutically effective amount of a composition, small molecule, peptide, vaccine, antibody, vector construct, viral vector, cell, or therapeutic agent of the present disclosure may be readily determined by one of ordinary skill by routine methods known in the art. Dosage regimen may be adjusted to provide the optimum therapeutic response.

As used herein, the terms "increasing" and "decreasing" refer to modulating resulting in, respectively, greater or lesser amounts, of function, expression, or activity of a metric relative to a reference. For example, subsequent to administration of a phosphatase inhibitor in a method described herein, the amount of a marker of a metric (e.g., the level of a phosphoneoantigen and/or the activity and/or expression level of a phophatase) as described herein may be increased or decreased in a subject by at least 1%, 2%, 5%, 10%, 15%, 20%, 25%, 30%, 35%, 40%, 45%, 50%, 55%, 60%, 65%, 70%, 75%, 80%, 85%, 90%, 95%, 100% or more relative to the amount of the marker prior to administration. Generally, the metric is measured after administration at a time that the administration has had the recited effect, e.g., at least 6 hours, 12 hours, 24 hours, one week, one month, 3 months, or 6 months, after a treatment regimen has begun.

As used herein, a "pharmaceutical composition" or "pharmaceutical preparation" is a composition or preparation having pharmacological activity or other direct effect in the mitigation, treatment, or prevention of disease, and/or a finished dosage form or formulation thereof and which is indicated for use in a subject (e.g., for use in a human subject).

As used herein, the term "pharmaceutically acceptable" refers to those compounds, materials, compositions and/or dosage forms, which are suitable for contact with the tissues of a subject, such as a mammal (e.g., a human) without excessive toxicity, irritation, allergic response and other problem complications commensurate with a reasonable benefit/risk ratio.

As used herein, the terms "subject" and "patient" refer to an animal (e.g., a mammal, such as a human). A subject to be treated according to the methods described herein may be one who has been diagnosed with a particular condition, or one at risk of developing such conditions. Diagnosis may be performed by any method or technique known in the art. One skilled in the art will understand that a subject to be treated according to the present disclosure may have been subjected to standard tests or may have been identified, without examination, as one at risk due to the presence of one or more risk factors associated with the disease or condition.

"Treatment" and "treating," as used herein, refer to the medical management of a subject with the intent to improve, ameliorate, stabilize (i.e., not worsen), prevent or cure a disease, pathological condition, or disorder. This term includes active treatment (treatment directed to improve the disease, pathological condition, or disorder), causal treatment (treatment directed to the cause of the associated disease, pathological condition, or disorder), palliative treatment (treatment designed for the relief of symptoms), preventative treatment (treatment directed to minimizing or partially or completely inhibiting the development of the associated disease, pathological condition, or disorder); and supportive treatment (treatment employed to supplement another therapy). Treatment also includes diminishment of the extent of the disease or condition; preventing spread of the disease or condition; delay or slowing the progress of the disease or condition; amelioration or palliation of the disease or condition; and remission (whether partial or total), whether detectable or undetectable. "Ameliorating" or "palliating" a disease or condition means that the extent and/or undesirable clinical manifestations of the disease, disorder, or condition are lessened and/or time course of the progression is slowed or lengthened, as compared to the extent or time course in the absence of treatment. "Treatment" can also mean prolonging survival as compared to expected survival if not receiving treatment. Those in need of treatment include those already with the condition or disorder, as well as those prone to have the condition or disorder or those in which the condition or disorder is to be prevented.

As used herein, the term "therapeutic agent" refers to any agent administered to a subject or used a cell that produces a treatment effect, as described herein. A therapeutic agent may include any composition, small molecule, peptide, vaccine, antibody, vector construct, viral vector, or cell of the present disclosure. In preferred embodiments, the therapeutic agent is administered in an amount that results in a beneficial or desired result in a subject as compared to a control. One or more therapeutic agents described herein (e.g., a phosphatase inhibitor, an immune checkpoint modulator, an immunomodulatory cytokine, an agent that inhibits myeloid derived suppressor cells, an anti-proliferative agent, a phosphoneoantigen vaccine, or CAR-T) may be administered as a monotherapy or in combination as described herein.

As used herein, the term "cancer" refers to a condition characterized by unregulated or abnormal cell growth. The terms "cancer cell," "tumor cell," and "tumor" refer to an abnormal cell, mass, or population of cells that result from excessive division that may be malignant or benign and all pre-cancerous and cancerous cells and tissues.

As used herein, the term "activation" refers to the response of an immune cell to a perceived insult. When immune cells become activated, they proliferate, secrete pro-inflammatory cytokines, differentiate, present antigens, become more polarized, and become more phagocytic and cytotoxic. Factors that stimulate immune cell activation include pro-inflammatory cytokines, pathogens, and non-self antigen presentation (e.g., antigens from pathogens presented by dendritic cells, macrophages, or B cells).

As used herein, the term "modulating an immune response" refers to any alteration in a cell of the immune system or any alteration in the activity of a cell involved in the immune response. Such regulation or modulation includes an increase or decrease in the number of various cell types, an increase or decrease in the activity of these cells, or any other changes that can occur within the immune system. Cells involved in the immune response include, but are not limited to, T lymphocytes (T cells), B lymphocytes (B cells), natural killer (NK) cells, ILCs, macrophages, eosinophils, mast cells, dendritic cells and neutrophils. In some cases, "modulating" the immune response means the immune response is stimulated or enhanced, and in other cases "modulating" the immune response means suppression of the immune system.

As used herein, the term "T cell" refers to a type of lymphocyte that plays a central role in cell-mediated immunity. T cells can be distinguished from other lymphocytes, such as B cells and natural killer cells, by the presence of a T-cell receptor (TCR) on the cell surface. There are several subsets of T cells, each having a distinct function (e.g., effector T cells and memory T cells).

As used herein, the term "helper T cell" (Th cells) refers to a subset of T cell that assists other white blood cells in immunologic processes, including maturation of B cells into plasma cells and memory B cells, and activation of cytotoxic T cells and macrophages. These cells are also known as CD4+ T cells because they express the CD4 glycoprotein on their surfaces. Th cells become activated when they are presented with peptide antigens by MHC class II molecules, which are expressed on the surface of antigen presenting cells (APCs). Once activated, they divide rapidly and secrete cytokines that regulate or assist in the active immune response. Th cells can differentiate into one of several subtypes (e.g., Th1, Th2, Th3, Th17, or TH9), which secrete different cytokines to facilitate different types of immune responses.

As used herein, the term "regulatory T cells" (Tregs) refers to a subpopulation of immunosuppressive T cells expressing the biomarkers CD4, FOXP3, and CD25. Tregs modulate the immune system, maintain tolerance to self-antigens, prevent autoimmune disease, and also suppress the anti-tumor immune response. Tregs are thought to suppress tumor immunity, thus hindering the body's innate ability to control the growth of cancerous cells. Tregs execute their immunosuppressive effects through IL-2/IL-2 receptor-dependent and CTLA-4-dependent mechanisms, and by production of inhibitory cytokines (e.g., IL-10 and TGF-beta).

As used here, the term "phosphoneoantigen" refers to any phosphorylated tumor antigen (e.g., a phosphorylated peptide or protein on the surface of a cell) which does not occur in a non-cancerous cell of the same type. Phosphoneoantigens may be derived from deregulated oncogenic cell signaling processes, for example, increased expression of a cancer-associated kinase in a cancer cell relative to a non-cancerous cell of the same type.

As used herein, the term "cancer-associated phosphatase" refers to any phosphatase which is selectively expressed or overexpressed in a cancer cell relative to a wild-type cell of the same type. Expression of a cancer-associated phosphatase may occur as a result of a genetic mutation associated with cancer. A cancer-associated phosphatase may be a phosphatase that is typically only expressed during development (e.g., in a fetal of placental cell) and which has been reactivated due to genetic mutation associated with cancer. Cancer associated phosphatases described herein include alkaline phosphatases (e.g., ALPP, ALPP-L2, and ALPL), acid phosphatases (e.g., ACPP), and protein serine/threonine phosphatases (PSP), as described herein.

As used herein, the term "immune checkpoint modulator" refers to immunotherapy agents used in the treatment of cancer to modulate the immune system. Immune checkpoint modulators (e.g., checkpoint inhibitors) can be broken down into at least 4 major categories: i) agents such as antibodies that block an inhibitory pathway directly on T cells or NK cells (e.g., PD-1 targeting antibodies such as nivolumab and pembrolizumab, antibodies targeting TIM-3, and antibodies targeting LAG-3, 2B4, CD160, A2aR, BTLA, CGEN-15049, or KIR), ii) agents such as antibodies that activate stimulatory pathways directly on T cells or NK cells (e.g., antibodies targeting OX40, GITR, or 4-1BB), iii) agents such as antibodies that block a suppressive pathway on immune cells or rely on antibody-dependent cellular cytotoxicity to deplete suppressive populations of immune cells (e.g., CTLA-4 targeting antibodies such as ipilimumab, antibodies targeting VISTA, and antibodies targeting PD-L2, Gr1, or Ly6G), and iv) agents such as antibodies that block a suppressive pathway directly on cancer cells or that rely on antibody-dependent cellular cytotoxicity to enhance cytotoxicity to cancer cells (e.g., rituximab, antibodies targeting PD-L1, and antibodies targeting B7-H3, B7-H4, Gal-9, or MUC1). Exemplary immune checkpoint modulators are provided herein.

As used herein, the term "immunomodulatory cytokine" refers to a small protein involved in cell signaling. Immunomodulatory cytokines can be produced and secreted by immune cells, such as T cells, B cells, macrophages, and mast cells, and include chemokines, interferons, interleukins, lymphokines, and tumor necrosis factors. Exemplary immunomodulatory cytokines are provided herein.

As used herein, the term "agent that inhibits myeloid derived suppressor cells" refers to any therapeutic agent that inhibits the function of myeloid derived suppressor cells, e.g., deactivates myeloid derived suppressor cells, differentiates myeloid derived suppressor cells into mature cells (fully differentiated myeloid derived suppressor cells are less immunosuppressive than early myeloid cells), blocks the development of myeloid derived suppressor cells, or decreases the number of myeloid derived suppressor cells in a subject. Agents that inhibit myeloid derived suppressor cells are known to those of skill in the art and may include small molecule inhibitors of myeloid derived suppressor cells, antibodies, or therapeutic nucleic acids. Agents that inhibit myeloid derived suppressor cells include, but are not limited to, indoleamine 2,3-dioxygenase (IDO) inhibitors, anti-VEGF antibodies, nitric oxide synthetase (NOS) inhibitors, CSF-1R inhibitors, arginase inhibitors, or multi-kinase inhibitors. Other exemplary agents that inhibit myeloid derived suppressor cells are provided in Wesolowski et al., J Immunother Cancer. 1:10 (2013), which is incorporated herein by reference in its entirety.

Other features and advantages of the invention will be apparent from the following description of the preferred embodiments thereof, and from the claims.

### Detailed Description of the Invention

We describe herein methods and compositions for increasing tumor antigenicity by increasing the level of expression of phosphoneoantigens in cells (e.g., restoring the expression of a phosphoneoantigen on the surface of a cancer cell). The invention also provides methods and compositions for enhancing recognition of phosphoneoantigens by immune cells. Below we also describe methods and compositions for the treatment of cancer by increasing tumor antigenicity and/or enhancing recognition of phosphoneoantigens by immune cells.

### Phosphoneoantigens

Genetic mutations accumulated in cancer include both driver mutations that alter oncogenic signaling pathways and passenger mutations that do not contribute to cancer. Although these passenger mutations do not lead to oncogenesis, a small proportion can become recognized by the immune system as neo-antigens. Neo-antigens may be recognized by the immune system, and thus tumor-specific neo-antigens may enable targeted cell killing of tumor cells by the immune system.

In addition to protein residue changes, neo-antigens may be generated through alterations to posttranslational modification of proteins. For example, genetic mutations that accumulate in cancer may give rise to phosphoneoantigens (e.g., cancer-specific phosphorylation of proteins on the surface of cancer cells) that may be recognized by the immune system.

Phosphoneoantigens are a subset of phosphorylated tumor antigens derived from deregulated oncogenic cell signaling processes that do not naturally occur. This abnormal phosphorylation creates "altered self' and when the phosphoprotein is degraded, the peptide fragments retain the phosphate group, marking them as abnormal.

Secreted phosphatases are able to remove phosphate groups from peptides. The present invention is based on the discovery that many cancers evolve to "turn-on" (increase the expression or activity of) cancer-associated phosphatases, for example placental phosphatases, to strip phosphopeptides of their phosphate groups, thus reducing overall tumor antigenicity.

Inhibition of cancer-associated phosphatase activity therefore prevents the destruction of phosphoneoantigens and enables detection of cancer cells by the immune response. Specific inhibitors of these enzymes restores phosphorylated tumor antigens, enhances recognition of tumors by T-cells, and expands activity of immuno-oncology agents such as those described herein (e.g., checkpoint modulators, immunomodulatory cytokines, or agents that inhibit myeloid derived suppressor cells).

### Cancer-associated phosphatases

Cancer-associated phosphatases described herein include any phosphatase which is selectively expressed or overexpressed in a cancer cell relative to a wild-type cell of the same type. Expression of a cancer-associated phosphatase may occur as a result of a genetic mutation associated with cancer. A cancer-associated phosphatase may be a phosphatase that is typically only expressed during development (e.g., in a fetal of placental cell) and which has been reactivated due to genetic mutation associated with cancer. Expression of a cancer-associated phosphatase may result in dephosphorylation of a phosphoneoantigen, thus reducing the tumor antigenicity of the cancer cell.

### Alkaline phosphatases

Exemplary cancer-associated phosphatases useful in the invention include alkaline phosphatases. There are at least four distinct but related human alkaline phosphatases: intestinal (ALPI), placental (ALPP), placental-like (ALPPL2), and tissue-nonspecific (ALPL) (primarily expressed in liver, bone, and kidney). Alkaline phosphatases contain zinc ions and magnesium ions that geometrically coordinate with the substrate phosphate monoester moiety mediating the hydrolysis. Owing to this mechanism, they have very broad catalytic activity against phosphate groups bound by both nucleic acids and proteins. Their high catalytic activity has led to wide use in biomedical research to dephosphorylate proteins and nucleic acids and, linked to antibodies, as detection agents in immunoassays such as ELISA and immunohistochemistry.

Alkaline phosphatase, placental type (ALPP; Gene name: ALPP; UniProt P05187) exhibits highly restricted expression primarily expressed in placenta. The exact biological role for the enzyme has not been elucidated, but biochemically, ALPP is the most thermostable alkaline phosphatase and relatively resistant to chemical inhibition. Expression of ALPP in healthy and cancerous tissues is provided in Figs. 1A-C.

Alkaline phosphatase, placental-like 2 (ALPP-L2; Gene name: ALPPL2; UniProt P10696) shows 97.3% sequence homology with ALPP and exhibits identical catalytic and biological activity of ALPP. Expression of ALPP-L2 in healthy and cancerous tissues is provided in Figs. 2A-C.

The ALPP and ALPP-L2 enzymes are not known to be involved in any physiological process outside of the placenta. Thus, targeting these enzymes may provide increased safety and tolerability relative to existing immune tolerance mechanisms such as PD-1/PD-L1 and regulatory T-cell mediated tolerance that are known to be co-opted into cancer immune-evasion. Inhibition of either pathway is known to be associated with severe autoimmune-like toxicities, and identifies these processes as being critical for self-tolerance.

Alkaline phosphatase, intestinal (ALPI; Gene name: ALPI; UniProt P09923) encodes a digestive brush-border enzyme. This enzyme is highly expressed in intestine and also upregulated in colorectal cancer, renal cancer and liver cancer. Expression of ALPI in healthy and cancerous tissues is provided in Figs. 3A-C.

### Acid phosphatases

Other useful cancer-associated phosphatases include acid phosphatases. There are multiple acid phosphatase isoenzymes that vary by tissue type, including ACP1, ACP2, ACPP (i.e. ACP3), ACP5, ACP6, and ACPT.

Prostatic acid phosphatase (ACPP, also termed PAP or PSAP) is exclusively expressed in prostate tissue. Prostate cancer also exhibits very high expression of ACPP. Expression of ACPP in healthy and cancerous tissues is provided in Figs. 4A-C.

### Protein serine/threonine phosphatases

Cancer-associated phosphatases are also useful and include, without limitation, protein serine/threonine phosphatases (PSPs). PSPs are phosphoprotein phosphatases that acts upon phosphorylated serine/threonine residues. There are multiple known groups of PSPs including PPP1 (α, β, γ1, γ2), PPP2 (formerly 2A), PPP3 (formerly 2b, also known as calcineurin), PPP2C, PPP4, PPP5, and PPP6.

### Inhibitors of cancer-associated phosphatases

Inhibitors of cancer-associated phosphatases as described herein typically reduce or inhibit phosphatase expression, function or signaling in order to treat cancer (e.g., by increasing antigenicity of a cancer cell). In preferred embodiments, the inhibitor of a cancer-associated phosphatase is a specific inhibitor of the cancer-associated phosphatase associated with the cancer being treated.

An inhibitor of a cancer-associated phosphatase can be selected from a number of different modalities. An inhibitor of a cancer-associated phosphatase can be a nucleic acid molecule (e.g., DNA molecule or RNA molecule, e.g., mRNA, or a hybrid DNA-RNA molecule), a polypeptide (e.g., an antibody molecule, e.g., an antibody or antigen binding fragment thereof), a nuclease (e.g., Cas9, TALEN, or ZFN), or a small molecule (e.g., a small molecule antagonist of a cancer-associated phosphatase). An inhibitor of a cancer-associated phosphatase can also be a viral vector expressing an inhibitor of a cancer-associated phosphatase or a cell infected with a viral vector.

### Small molecules

In preferred embodiments, the inhibitor of the cancer-associated phosphatase is a small molecule inhibitor, most preferably a small molecule inhibitor selective for the cancer-associated phosphatase (e.g., ALPP, ALPP-L2, ALPI, ALPL, ACPP, or PSP). Exemplary small molecule inhibitors of cancer-associated phosphatases include ML095, thiophosphate, L-p-bromotetramisole, tetramisole, levamisole, 5804079, L-phenylalanine, 1-paphthyl phosphate, MLS-0315848, MLS0390945, and MLS-0315687 (CID-715454). Table 1 provides the inhibition constant (Ki or IC₅₀) for these exemplary phosphatase inhibitors ("+" indicates that inhibitory activity has been observed, but that a Ki or IC50 has not been quantified).

**Table 1.**

| **Phosphatase inhibitor** | **ALPP** | **ALPI** | **ALPL** |
|---|---|---|---|
| Thiophosphate | Ki=7.2uM | N/A | Ki=30uM |
| L-p-bromotetramisole | Ki=18uM | N/A | Ki=56uM |
| Tetramisole | + | + | IC₅₀=23.2uM |
| Levamisole | >400uM | >400uM | Ki=21uM |
| | | | IC₅₀=23.2uM |
| 5804079 | N/A | N/A | Ki=6.5uM |
| L-Phenylalanine (RPMI = 15mg/L = 90uM) | IC₅₀=19 mM | IC₅₀=3mM | IC₅₀=19mM |
| 1-Naphthyl phosphate | + | + | + |
| ML095 | IC₅₀=1.9uM | IC₅₀=50uM | IC₅₀=7.2uM |
| MLS-0315848 | IC50=3.7uM | IC₅₀>100uM | IC₅₀>100uM |
| MLS-0390945 | IC₅₀=2.1 uM | IC₅₀=53uM | IC₅₀>100uM |
| MLS-0315687 (CID-715454) | IC₅₀=1.2uM | IC₅₀=49uM | IC₅₀=5.6uM |

Additional small molecule inhibitors of cancer-associated phosphatases are known to those of skill in the art. Small molecule inhibitors of cancer-associated phosphatases can also be identified through screening based on their ability to reduce or inhibit the activity or expression of the cancer-associated phosphatase. Small molecules include, but are not limited to, small peptides, peptidomimetics (e.g., peptoids), amino acids, amino acid analogs, synthetic polynucleotides, polynucleotide analogs, nucleotides, nucleotide analogs, organic and inorganic compounds (including heterorganic and organometallic compounds) generally having a molecular weight less than about 5,000 grams per mole, e.g., organic or inorganic compounds having a molecular weight less than about 2,000 grams per mole, e.g., organic or inorganic compounds having a molecular weight less than about 1,000 grams per mole, e.g., organic or inorganic compounds having a molecular weight less than about 500 grams per mole, and salts, esters, and other pharmaceutically acceptable forms of such compounds.

A pharmaceutical composition including a small molecule inhibitor of a cancer-associated phosphatase can be formulated for treatment of cancer as described herein.

### Antibodies

An inhibitor of a cancer-associated phosphatase can be an antibody or antigen binding fragment thereof. For example, an inhibitor of a cancer-associated phosphatase described herein is an antibody that reduces or blocks the activity and/or function of the cancer-associated phosphatase through binding to the cancer-associated phosphatase to block the binding between the cancer-associated phosphatase and a binding partner.

### Nucleic acids

In some embodiments, the inhibitor of a cancer-associated phosphatase is an inhibitory RNA molecule, e.g., that acts by way of the RNA interference (RNAi) pathway. An inhibitory RNA molecule can decrease the expression level (e.g., protein level or mRNA level) of a cancer-associated phosphatase. For example, an inhibitory RNA molecule includes a short interfering RNA, short hairpin RNA, and/or a microRNA that targets a cancer-associated phosphatase. An siRNA is a double-stranded RNA molecule that typically has a length of about 19-25 base pairs. A shRNA is a RNA molecule including a hairpin turn that decreases expression of target genes via RNAi. shRNAs can be delivered to cells in the form of plasmids, e.g., viral or bacterial vectors, e.g., by transfection, electroporation, or transduction). A microRNA is a non-coding RNA molecule that typically has a length of about 22 nucleotides. miRNAs bind to target sites on mRNA molecules and silence the mRNA, e.g., by causing cleavage of the mRNA, destabilization of the mRNA, or inhibition of translation of the mRNA. In embodiments, the inhibitory RNA molecule decreases the level and/or activity of a negative regulator of function or a positive regulator of function. In other embodiments, the inhibitory RNA molecule decreases the level and/or activity of an inhibitor of a positive regulator of function.

An inhibitory RNA molecule can be modified, e.g., to contain modified nucleotides, e.g., 2'-fluoro, 2'-o-methyl, 2'-deoxy, unlocked nucleic acid, 2'-hydroxy, phosphorothioate, 2'-thiouridine, 4'-thiouridine, 2'-deoxyuridine. Without being bound by theory, it is believed that certain modification can increase nuclease resistance and/or serum stability, or decrease immunogenicity.

In some embodiments, the inhibitory RNA molecule decreases the level and/or activity or function of a cancer-associated phosphatase. In other embodiments, the inhibitor RNA molecule increases degradation of a cancer-associated phosphatase and/or decreases the stability of a cancer-associated phosphatase. The inhibitory RNA molecule can be chemically synthesized or transcribed in vitro.

The making and use of inhibitory therapeutic agents based on non-coding RNA such as ribozymes, RNAse P, siRNAs, and miRNAs are also known in the art, for example, as described in Sioud, RNA Therapeutics: Function, Design, and Delivery (Methods in Molecular Biology). Humana Press 2010.

### Viral vectors

The cancer-associated phosphatase inhibitor can be delivered by a viral vector (e.g., a viral vector expressing a cancer-associated phosphatase inhibitor). Viral vectors can be directly administered (e.g., injected) to a tumor to treat cancer.

Viral genomes provide a rich source of vectors that can be used for the efficient delivery of exogenous genes into a mammalian cell. Viral genomes are particularly useful vectors for gene delivery because the polynucleotides contained within such genomes are typically incorporated into the nuclear genome of a mammalian cell by generalized or specialized transduction. These processes occur as part of the natural viral replication cycle, and do not require added proteins or reagents in order to induce gene integration. Examples of viral vectors include a retrovirus (e.g., Retroviridae family viral vector), adenovirus (e.g., Ad5, Ad26, Ad34, Ad35, and Ad48), parvovirus (e.g., adeno-associated viruses), coronavirus, negative strand RNA viruses such as orthomyxovirus (e.g., influenza virus), rhabdovirus (e.g., rabies and vesicular stomatitis virus), paramyxovirus (e.g., measles and Sendai), positive strand RNA viruses, such as picornavirus and alphavirus, and double stranded DNA viruses including adenovirus, herpesvirus (e.g., Herpes Simplex virus types 1 and 2, Epstein-Barr virus, cytomegalovirus, replication deficient herpes virus), and poxvirus (e.g., vaccinia, modified vaccinia Ankara (MVA), fowlpox and canarypox). Other viruses include Norwalk virus, togavirus, flavivirus, reoviruses, papovavirus, hepadnavirus, human papilloma virus, human foamy virus, and hepatitis virus, for example. Examples of retroviruses include: avian leukosis-sarcoma, avian C-type viruses, mammalian C-type, B-type viruses, D-type viruses, oncoretroviruses, HTLV-BLV group, lentivirus, alpharetrovirus, gammaretrovirus, spumavirus (Coffin, J. M., Retroviridae: The viruses and their replication, Virology (Third Edition) Lippincott-Raven, Philadelphia, 1996). Other examples include murine leukemia viruses, murine sarcoma viruses, mouse mammary tumor virus, bovine leukemia virus, feline leukemia virus, feline sarcoma virus, avian leukemia virus, human T-cell leukemia virus, baboon endogenous virus, Gibbon ape leukemia virus, Mason Pfizer monkey virus, simian immunodeficiency virus, simian sarcoma virus, Rous sarcoma virus, and lentiviruses.

### Combination therapies

An inhibitor of a cancer-associated phosphatase described herein can be administered in combination with a second therapeutic agent for treatment of cancer. In some embodiments, the second therapeutic agent is selected based on tumor type, tumor tissue of origin, tumor stage, or mutations in genes expressed by the tumor.

### Immune checkpoint modulators

In a preferred embodiment of the invention, an inhibitor of a cancer-associated phosphatase is administered in combination with an immune checkpoint modulator. In some embodiments, the immune checkpoint modulator is administered at a lower dose when administered in combination with the inhibitor of a cancer-associated phosphatase than when administered in the absence of the inhibitor of a cancer-associated phosphatase (e.g., a dose of 1%, 2%, 5%, 10%, 15%, 20%, 30%, 40%, 50%, 60%, 70%, 80%, 90%, or 100% or less than when administered in the administered in the absence of an inhibitor of a cancer-associated phosphatase). In some embodiments, the therapeutic activity of the immune checkpoint modulator is enhanced when administered in combination with an inhibitor of a cancer-associated phosphatase.

There are at least four types of immune checkpoint modulators (also known as checkpoint inhibitors): i) agents such as antibodies that block an inhibitory pathway directly on T cells or NK cells (e.g., PD-1 targeting antibodies such as nivolumab and pembrolizumab, antibodies targeting TIM-3, and antibodies targeting LAG-3, 2B4, CD160, A2aR, BTLA, CGEN-15049, or KIR), ii) agents such as antibodies that activate stimulatory pathways directly on T cells or NK cells (e.g., antibodies targeting OX40, GITR, or 4-1BB), iii) agents such as antibodies that block a suppressive pathway on immune cells or rely on antibody-dependent cellular cytotoxicity to deplete suppressive populations of immune cells (e.g., CTLA-4 targeting antibodies such as ipilimumab, antibodies targeting VISTA, and antibodies targeting PD-L2, Gr1, or Ly6G), and iv) agents such as antibodies that block a suppressive pathway directly on cancer cells or that rely on antibody-dependent cellular cytotoxicity to enhance cytotoxicity to cancer cells (e.g., rituximab, antibodies targeting PD-L1, and antibodies targeting B7-H3, B7-H4, Gal-9, or MUC1). Such agents described herein can be designed and produced, e.g., by conventional methods known in the art (e.g., Templeton, Gene and Cell Therapy, 2015; Green and Sambrook, Molecular Cloning, 2012).

Exemplary useful immune checkpoint inhibitors include an anti-PD-1 antibody, an anti-CTLA-4 antibody, an anti-TIM-3 antibody, an anti-NKG2A antibody, an anti-LAG-3 antibody, an anti-cMet antibody, an anti-CTLA-4/PD-1 bispecific antibody, an anti-TIM-3/PD-1 bispecific antibody, an anti-PD1/LAG3 bispecific antibody, an anti-PD-1/cMet bispecific antibody, or an antigen-binding fragment thereof.

Further exemplary immune checkpoint inhibitors include pembrolizumab, nivolumab, atelizumab, spartalizumab, camrelizumab, pidilizumab, cemiplimab, tislelizumab, JS001, MEDI0680, BCD-100, JNJ-63723283, CBT-501, TSR-042, MGA012, PF-06801591, KN035, LZM009, XmAb20717, AGEN2034, Sym021, AK105, AK104, HLX10, CS1003, STI-1110, MGD013, MCLA-134, AM001, or EDI5752.

### Immunomodulatory cytokines

In a preferred embodiment, an inhibitor of a cancer-associated phosphatase is administered in combination with an immunomodulatory cytokine. For example, the immunomodulatory cytokine is administered at a lower dose when administered in combination with the inhibitor of a cancer-associated phosphatase than when administered in the absence of the inhibitor of a cancer-associated phosphatase (e.g., a dose of 1%, 2%, 5%, 10%, 15%, 20%, 30%, 40%, 50%, 60%, 70%, 80%, 90%, or 100% or less than when administered in the administered in the absence of an inhibitor of a cancer-associated phosphatase). In another example, the therapeutic activity of the immunomodulatory cytokine is enhanced when administered in combination with an inhibitor of a cancer-associated phosphatase.

Immunomodulatory cytokines are small proteins involved in cell signaling. Immunomodulatory cytokines can be produced and secreted by immune cells, such as T cells, B cells, macrophages, and mast cells, and include chemokines, interferons, interleukins, lymphokines, and tumor necrosis factors.

Immunomodulatory cytokines described herein include anti-inflammatory cytokines produced or secreted by an immune cell that reduces inflammation. Immune cells that produce and secrete anti-inflammatory cytokines include T cells (e.g., Th cells) macrophages, B cells, and mast cells. Anti-inflammatory cytokines include IL4, IL-10, IL-11, IL-13, interferon alpha (IFNα) and transforming growth factor-beta (TGFβ).

Additional immunomodulatory cytokines include small chemokines that can induce directed chemotaxis in nearby cells. Exemplary classes of such chemokines include CC chemokines, CXC chemokines, C chemokines, and CX3C chemokines. Chemokines can regulate immune cell migration and homing, including the migration and homing of monocytes, macrophages, T cells, mast cells, eosinophils, and neutrophils. Chemokines responsible for immune cell migration include CCL19, CCL21, CCL14, CCL20, CCL25, CCL27, CXCL12, CXCL13, CCR9, CCR10, and CXCR5. Chemokines that can direct the migration of inflammatory leukocytes to sites of inflammation or injury include CCL2, CCL3, CCL5, CXCL1, CXCL2, and CXCL8.

Preferably such an immunomodulatory cytokine is selected from an interleukin or an interferon such as IL-2, IL-12, IL-15, IFNα, IFNβ, IFNγ, or a synthetic mimic of a naturally-occurring cytokine (e.g., Neo-2/15, which mimics IL-2 and IL-15 activity).

### Agents that inhibit myeloid derived suppressor cells (MDSCs)

In a preferred embodiment, an inhibitor of a cancer-associated phosphatase is administered in combination with an agent that inhibits myeloid derived suppressor cells. For example, the agent that inhibits myeloid derived suppressor cells is administered at a lower dose when administered in combination with the inhibitor of a cancer-associated phosphatase than when administered in the absence of the inhibitor of a cancer-associated phosphatase (e.g., a dose of 1%, 2%, 5%, 10%, 15%, 20%, 30%, 40%, 50%, 60%, 70%, 80%, 90%, or 100% or less than when administered in the administered in the absence of an inhibitor of a cancer-associated phosphatase). In another example, the therapeutic activity of the agent that inhibits myeloid derived suppressor cells is enhanced when administered in combination with an inhibitor of a cancer-associated phosphatase.

Myeloid derived suppressor cells are a heterogeneous population of immature myeloid cells that are increased in states of cancer, inflammation and infection. In malignant states, myeloid derived suppressor cells are induced by tumor secreted growth factors. Myeloid derived suppressor cells play an important part in suppression of host immune responses through several mechanisms such as production of arginase 1, release of reactive oxygen species and nitric oxide and secretion of immune-suppressive cytokines. This leads to a permissive immune environment necessary for the growth of malignant cells. Myeloid derived suppressor cells may also contribute to angiogenesis and tumor invasion.

Agents that inhibit myeloid derived suppressor cells are known to those of skill in the art and may include small molecule inhibitors of myeloid derived suppressor cells, antibodies, or therapeutic nucleic acids. Agents that inhibit myeloid derived suppressor cells include indoleamine 2,3-dioxygenase (IDO) inhibitors, anti-VEGF antibodies, nitric oxide synthetase (NOS) inhibitors, CSF-1R inhibitors, arginase inhibitors, or multi-kinase inhibitors. Other exemplary agents that inhibit myeloid derived suppressor cells are provided in Wesolowski et al., J Immunother Cancer. 1:10 (2013), which is incorporated herein by reference in its entirety.

### Anti-proliferative agents

One or more anti-proliferative agents (e.g., one or more chemotherapeutic agents) may be administered in combination with an inhibitor of a cancer-associated phosphatase. Anti-proliferative agents include alkylating agents, antimetabolites, folic acid analogs, pyrimidine analogs, purine analogs and related inhibitors, vinca alkaloids, epipodopyyllotoxins, antibiotics, L-asparaginase, topoisomerase inhibitors, interferons, platinum coordination complexes, anthracenedione substituted urea, methyl hydrazine derivatives, adrenocortical suppressant, adrenocorticosteroides, progestins, estrogens, antiestrogen, androgens, antiandrogen, and gonadotropin-releasing hormone analog. Also included is 5-fluorouracil (5-FU), leucovorin (LV), irenotecan, oxaliplatin, capecitabine, paclitaxel and doxetaxel. Nonlimiting examples of chemotherapeutic agents include alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g., calicheamicin, especially calicheamicin gammall and calicheamicin omegall; dynemicin, including dynemicin A; bisphosphonates, such as clodronate; an esperamicin; as well as neocarzinostatin chromophore and related chromoprotein enediyne antiobiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfomithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine;; razoxane; rhizoxin; sizofuran; spirogermanium; tenuazonic acid; triaziquone; 2,2',2"-trichlorotriethylamine; trichothecenes (especially T-2 toxin, verracurin A, roridin A, and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., , paclitaxel; chloranbucil; gemcitabine; 6-thioguanine; mercaptopurine; methotrexate; platinum coordination complexes such as cisplatin, oxaliplatin and carboplatin; vinblastine; platinum; etoposide (VP-16); ifosfamide; mitoxantrone; vincristine; vinorelbine; novantrone; teniposide; edatrexate; daunomycin; aminopterin; xeloda; ibandronate; irinotecan (e.g., CPT-11); topoisomerase inhibitor RFS 2000; difluoromethylornithine (DMFO); retinoids such as retinoic acid; capecitabine; and pharmaceutically acceptable salts, acids or derivatives of any of the above. Two or more anti-proliferative agents can be used in a cocktail to be administered in combination with the first therapeutic agent described herein. Suitable dosing regimens of combination chemotherapies are known in the art.

### Non-drug therapies

Another type of agent that can be administered in combination with an inhibitor of a cancer-associated phosphatase is a therapeutic agent that is a non-drug treatment. For example, the second therapeutic agent is radiation therapy, cryotherapy, hyperthermia and/or surgical excision of tumor tissue.

### Phosphoneoantigen vaccine

Also useful are in increasing immune recognition of cancer cells are methods of vaccinating a subject (e.g., a human subject having cancer or at risk of developing cancer) with a phosphoneoantigen and, optionally, a suitable adjuvant. Vaccination of a subject includes administering to the subject a phosphoneoantigen and, optionally, a suitable adjuvant, in an amount sufficient to induce an immune response against the phosphoneoantigen. The phosphoneoantigen may be a phosphoneoantigen described herein, such as a phosphoneoantigen associated with expression of overexpression of a cancer-associated phosphatase.

A phosphoneoantigen vaccine described herein may be administered to a subject as a monotherapy or as a combination therapy (e.g., as a combination therapy with an inhibitor of a cancer-associated phosphatase, an immunomodulatory cytokine, an agent that inhibits myeloid derived suppressor cells, an anti-proliferative agent, an immune checkpoint modulator, or any other therapeutic agent described herein). In preferred embodiments, the phosphoneoantigen vaccine is administered as a combination therapy with an inhibitor of a cancer-associated phosphatase.

Increasing the expression of the phosphoneoantigen in the cancer cell (e.g., by inhibition of the cancer-associated phosphatase) can enhance the immune response against the cancer cell upon vaccination with the phosphoneoantigen (e.g. enhances the immune response by 1%, 2%, 5%, 10%, 20%, 30%, 40%, 50%, 60%, 70%, 80%, 90%, 100%, 150%, 200%, 500%, or 1000% or more). In some embodiments, increasing the expression of the phosphoneoantigen in the cancer cell (e.g., by inhibition of the cancer-associated phosphatase) decreases the dose of the vaccination with the phosphoneoantigen required to achieve a therapeutic effect (e.g., decreases the dose by 1%, 2%, 5%, 10%, 20%, 30%, 40%, 50%, 60%, 70%, 80%, 90%, 100%, 150%, 200%, 500%, or 1000% or more).

Methods for treating cancer by administering an anti-cancer vaccine (e.g., a cancer vaccine including a tumor specific antigen) are known to those of skill in the art; see, for example, Buonaguro et al. Clin Vaccine Imunol. 18(1):23-34 (2011); and Tagliamonte et al. Hum Vaccin Immunother. 10(11):3332-3346 (2014). A phosphoneoantigen vaccine may include all or any portion of the phosphoneoantigen necessary to elicit an immune response specific to the phosphoneoantigen.

### Engineered T-cells

Another therapy that can be employed either as a monotherapy or a combination with the methods and compositions described herein is chimeric antigen receptor (CAR)-T therapy, or therapy with lymphocytes, such as autologous or allogeneic T cells, that have been modified to express a CAR that recognizes specific cancer antigens (e.g., a phosphoneoantigen). Commonly, CARs contain a single chain fragment variable (scFv) region of an antibody or a binding domain specific for a tumor associated antigen (TAA) coupled via hinge and transmembrane regions to cytoplasmic domains of T cell signaling molecules. The most common lymphocyte activation moieties include a T cell costimulatory domain (e.g., CD28 and/or CD137) in tandem with a T cell effector function triggering (e.g. CD3ξ) moiety. CARs have the ability to redirect T cell reactivity and specify toward a selected target in a non-MHC restricted manner, exploiting the antigen-binding properties of monoclonal antibodies. The non-MHC restricted antigen recognition gives CAR-T cells the ability to bypass a major mechanism of tumor escape.

The invention provides administering to a subject (e.g., a human subject having cancer or at risk of developing cancer) a T-cell that has been genetically modified to express a T-cell receptor (TCR), wherein the TCR binds specifically to a phosphoneoantigen.

Genetically modified T-cells may be administered to a subject as a monotherapy or as a combination therapy (e.g., as a combination therapy with an inhibitor of a cancer-associated phosphatase, an immunomodulatory cytokine, an agent that inhibits myeloid derived suppressor cells, an anti-proliferative agent, an immune checkpoint modulator, or any other therapeutic agent described herein). In preferred embodiments, genetically modified T-cells are administered as a combination therapy with an inhibitor of a cancer-associated phosphatase.

In some embodiments, increasing the expression of the phosphoneoantigen in the cancer cell (e.g., by inhibition of the cancer-associated phosphatase) enhances the immune response against the cancer cell upon administration of the genetically engineered T-cell (e.g. enhances the immune response by 1%, 2%, 5%, 10%, 20%, 30%, 40%, 50%, 60%, 70%, 80%, 90%, 100%, 150%, 200%, 500%, or 1000% or more). In some embodiments, increasing the expression of the phosphoneoantigen in the cancer cell (e.g., by inhibition of the cancer-associated phosphatase) decreases the dose of the genetically engineered T-cell required to achieve a therapeutic effect (e.g., decreases the dose by 1%, 2%, 5%, 10%, 20%, 30%, 40%, 50%, 60%, 70%, 80%, 90%, 100%, 150%, 200%, 500%, or 1000% or more).

### Modulation of immune cells

The methods described herein are useful for modulating the antigenicity of a cell and/or modulating an immune response in a subject or cell by administering to a subject or cell a therapeutic agent (e.g., an inhibitor of a cancer-associated phosphatase, optionally, in combination with a second therapeutic agent) in a dose (e.g., an effective amount) and for a time sufficient to modulate the antigenicity of the cell and/or the immune response of the subject. These methods can be used to treat a subject in need of modulating an immune response, e.g., a subject with cancer. One way to modulate an immune response is to modulate an immune cell activity. This modulation can occur in vivo (e.g., in a human subject or animal model) or in vitro (e.g., in acutely isolated or cultured cells, such as human cells from a patient, repository, or cell line, or rodent cells). The types of cells that can be modulated include T cells (e.g., peripheral T cells, cytotoxic T cells/CD8+ T cells, T helper cells/CD4+ T cells, memory T cells, regulatory T cells/Tregs, natural killer T cells/NKTs, mucosal associated invariant T cells, and gamma delta T cells), B cells (e.g., memory B cells, plasmablasts, plasma cells, follicular B cells/B-2 cells, marginal zone B cells, B-1 cells, regulatory B cells/Bregs), dendritic cells (e.g., myeloid DCs/conventional DCs, plasmacytoid DCs, or follicular DCs), granulocytes (e.g., eosinophils, mast cells, neutrophils, and basophils), monocytes, macrophages (e.g., peripheral macrophages or tissue resident macrophages or tumor-resident macrophages), myeloid-derived suppressor cells, natural killer (NK) cells, innate lymphoid cells (ILC1, ILC2, ILC3), thymocytes, and megakaryocytes.

The immune cell activities that can be modulated by administering to a subject or contacting a cell with an effective amount of a therapeutic agent described herein (e.g., an inhibitor of a cancer-associated phosphatase, optionally, in combination with a second therapeutic agent) include activation (e.g., macrophage, T cell, NK cell, ILC, B cell, dendritic cell, neutrophil, eosinophil, or basophil activation), phagocytosis (e.g., macrophage, neutrophil, monocyte, mast cell, B cell, eosinophil, or dendritic cell phagocytosis), antibody-dependent cell-mediated phagocytosis (e.g., ADCP by monocytes, macrophages, neutrophils, or dendritic cells), antibody-dependent cell-mediated cytotoxicity (e.g., ADCC by NK cells, ILCs, monocytes, macrophages, neutrophils, eosinophils, dendritic cells, or T cells), polarization (e.g., macrophage polarization toward an M1 or M2 phenotype or T cell polarization), proliferation (e.g., proliferation of B cells, T cells, monocytes, macrophages, dendritic cells, NK cells, ILCs, mast cells, neutrophils, eosinophils, or basophils), lymph node homing (e.g., lymph node homing of T cells, B cells, dendritic cells, or macrophages), lymph node egress (e.g., lymph node egress of T cells, B cells, dendritic cells, or macrophages), recruitment (e.g., recruitment of B cells, T cells, monocytes, ILCs, macrophages, dendritic cells, NK cells, mast cells, neutrophils, eosinophils, or basophils), migration (e.g., migration of B cells, T cells, monocytes, macrophages, dendritic cells, NK cells, ILCs, mast cells, neutrophils, eosinophils, or basophils), differentiation (e.g., regulatory T cell differentiation), immune cell cytokine production, antigen presentation (e.g., dendritic cell, macrophage, and B cell antigen presentation), maturation (e.g., dendritic cell maturation), and degranulation (e.g., mast cell, NK cell, ILCs, cytotoxic T cell, neutrophil, eosinophil, or basophil degranulation). Innervation of lymph nodes or lymphoid organs, development of high endothelial venules (HEVs), and development of ectopic or tertiary lymphoid organs (TLOs) can also be modulated using the methods described herein. Modulation can increase or decrease these activities, depending on the therapeutic agent (e.g., an inhibitor of a cancer-associated phosphatase, optionally, in combination with a second therapeutic agent) used to contact the cell or treat a subject.

In some embodiments, an effective amount of the therapeutic agent (e.g., an inhibitor of a cancer-associated phosphatase, optionally, in combination with a second therapeutic agent) is an amount sufficient to modulate (e.g., increase or decrease) one or more (e.g., 2 or more, 3 or more, 4 or more) of the following immune cell activities in the subject or cell: T cell polarization; T cell activation; dendritic cell activation; neutrophil activation; eosinophil activation; basophil activation; T cell proliferation; B cell proliferation; T cell proliferation; monocyte proliferation; macrophage proliferation; dendritic cell proliferation; NK cell proliferation; ILC proliferation; mast cell proliferation; neutrophil proliferation; eosinophil proliferation; basophil proliferation; cytotoxic T cell activation; circulating monocytes; peripheral blood hematopoietic stem cells; macrophage polarization; macrophage phagocytosis; macrophage ADCP, neutrophil phagocytosis; monocyte phagocytosis; mast cell phagocytosis; B cell phagocytosis; eosinophil phagocytosis; dendritic cell phagocytosis; macrophage activation; antigen presentation (e.g., dendritic cell, macrophage, and B cell antigen presentation); antigen presenting cell migration (e.g., dendritic cell, macrophage, and B cell migration); lymph node immune cell homing and cell egress (e.g., lymph node homing and egress of T cells, B cells, dendritic cells, or macrophages); NK cell activation; NK cell ADCC, mast cell degranulation; NK cell degranulation; ILC activation, ILC ADCC, ILC degranulation; cytotoxic T cell degranulation; neutrophil degranulation; eosinophil degranulation; basophil degranulation; neutrophil recruitment; eosinophil recruitment; NKT cell activation; B cell activation; regulatory T cell differentiation; dendritic cell maturation; development of HEVs; development of TLOs; or lymph node or secondary lymphoid organ innervation. In certain embodiments, the immune response (e.g., an immune cell activity listed herein) is increased or decreased in the subject or cell at least 1%, 2%, 5%, 10%, 15%, 20%, 25%, 30%, 35%, 40%, 50%, 60%, 70%, 80%, 100%, 150%, 200%, 300%, 400%, 500% or more, compared to before the administration. In certain embodiments, the immune response is increased or decreased in the subject or cell between 5-20%, between 5-50%, between 10-50%, between 20-80%, between 20-70%, between 50-200%, between 100%-500%.

After administration of a therapeutic agent (e.g., an inhibitor of a cancer-associated phosphatase, optionally, in combination with a second therapeutic agent) to treat a patient or contact a cell, a readout can be used to assess the effect on immune cell activity. Immune cell activity can be assessed by measuring a cytokine or marker associated with a particular immune cell type. In certain embodiments, the parameter is increased or decreased in the subject at least 1%, 2%, 5%, 10%, 15%, 20%, 25%, 30%, 35%, 40%, 50%, 60%, 70%, 80%, 100%, 150%, 200%, 300%, 400%, 500% or more, compared to before the administration. In certain embodiments, the parameter is increased or decreased in the subject between 5-20%, between 5-50%, between 10-50%, between 20-80%, between 20-70%, between 50-200%, between 100%-500%. A therapeutic agent (e.g., an inhibitor of a cancer-associated phosphatase, optionally, in combination with a second therapeutic agent) can be administered at a dose (e.g., an effective amount) and for a time sufficient to modulate an immune cell activity described herein below.

In another example, after a therapeutic agent (e.g., an inhibitor of a cancer-associated phosphatase, optionally, in combination with a second therapeutic agent) is administered to treat a patient or contact a cell, a readout can be used to assess the effect on immune cell migration. Immune cell migration can be assessed by measuring the number of immune cells in a location of interest (e.g., tumor, site of metastasis, lymph node, secondary lymphoid organ, tertiary lymphoid organ). Immune cell migration can also be assessed by measuring a chemokine, receptor, or marker associated with immune cell migration. In certain embodiments, the parameter is increased or decreased in the subject at least 1%, 2%, 5%, 10%, 15%, 20%, 25%, 30%, 35%, 40%, 50%, 60%, 70%, 80%, 100%, 150%, 200%, 300%, 400%, 500% or more, compared to before the administration. In certain embodiments, the parameter is increased or decreased in the subject between 5-20%, between 5-50%, between 10-50%, between 20-80%, between 20-70%, between 50-200%, between 100%-500%. A therapeutic agent (e.g., an inhibitor of a cancer-associated phosphatase, optionally, in combination with a second therapeutic agent) can be administered at a dose (e.g., an effective amount) and for a time sufficient to modulate an immune cell migration as described herein below.

A therapeutic agent (e.g., an inhibitor of a cancer-associated phosphatase, optionally, in combination with a second therapeutic agent) described herein can affect immune cell migration. Immune cell migration between peripheral tissues, the blood, and the lymphatic system as well as lymphoid organs is essential for the orchestration of productive innate and adaptive immune responses. Immune cell migration is largely regulated by trafficking molecules including integrins, immunoglobulin cell-adhesion molecules (IgSF CAMs), cadherins, selectins, and a family of small cytokines called chemokines. Cell adhesion molecules and chemokines regulate immune cell migration by both inducing extravasation from the circulation into peripheral tissues and acting as guidance cues within peripheral tissues themselves. For extravasation to occur, chemokines must act in concert with multiple trafficking molecules including C-type lectins (L-, P-, and E-selectin), multiple integrins, and cell adhesion molecules (ICAM-1, VCAM-1 and MAdCAM-1) to enable a multi-step cascade of immune cell capturing, rolling, arrest, and transmigration via the blood endothelial barrier. Some trafficking molecules are constitutively expressed and manage the migration of immune cells during homeostasis, while others are specifically upregulated by inflammatory processes such as autoimmunity and cancer.

In still other examples, a therapeutic agent (e.g., an inhibitor of a cancer-associated phosphatase, optionally, in combination with a second therapeutic agent) described herein increases one or more of T cell proliferation, T cell activation, T cell differentiation, or T cell cytokine production (e.g., T cell production of pro-inflammatory cytokines, e.g., IFNγ). In some embodiments, the T cell is an effector T cell, helper T cell, Th1 cell, Th2 cell, or Th17 cell. In some embodiments, a therapeutic agent (e.g., an inhibitor of a cancer-associated phosphatase, optionally, in combination with a second therapeutic agent) described herein increases inflammation.

### Cancer

The methods described herein can be used to treat cancer in a subject by administering to the subject an effective amount of a therapeutic agent (e.g., an inhibitor of a cancer-associated phosphatase, optionally, in combination with a second therapeutic agent) described herein. The method may include administering locally (e.g., intratumorally) to the subject a therapeutic agent (e.g., an inhibitor of a cancer-associated phosphatase, optionally, in combination with a second therapeutic agent) described herein in a dose (e.g., effective amount) and for a time sufficient to treat the cancer.

The methods described herein can also be used to potentiate or increase an immune response in a subject in need thereof, e.g., an anti-tumor immune response. For example, the subject has cancer, such as a cancer described herein. The methods described herein can also include a step of selecting a subject in need of potentiating an immune response, e.g., selecting a subject who has cancer or is at risk of developing cancer.

The therapeutic agent (e.g., an inhibitor of a cancer-associated phosphatase, optionally, in combination with a second therapeutic agent) can be administered in an amount sufficient to treat cancer. The therapeutic agent (e.g., an inhibitor of a cancer-associated phosphatase, optionally, in combination with a second therapeutic agent) can treat cancer by increasing cancer cell death in a subject (e.g., a human subject or animal model) or in a cancer cell culture (e.g., a culture generated from a patient tumor sample, a cancer cell line, or a repository of patient samples). A therapeutic agent (e.g., an inhibitor of a cancer-associated phosphatase, optionally, in combination with a second therapeutic agent) can increase cancer cell death by at least 1%, 2%, 5%, 10%, 15%, 20%, 25%, 30%, 35%, 40%, 50%, 60%, 70%, 80%, 90%, 95% or more compared to before administration to a subject or cancer cell culture. A therapeutic agent (e.g., an inhibitor of a cancer-associated phosphatase, optionally, in combination with a second therapeutic agent) can increase cancer cell death in a subject or cancer cell culture between 5-20%, between 5-50%, between 10-50%, between 20-80%, or between 20-70%.

The therapeutic agent (e.g., an inhibitor of a cancer-associated phosphatase, optionally, in combination with a second therapeutic agent) can also act to inhibit cancer cell growth, proliferation, metastasis, migration, or invasion, e.g., the method includes administering to the subject (e.g., a human subject or animal model) or a cancer cell culture (e.g., a culture generated from a patient tumor sample, a cancer cell line, or a repository of patient samples) a therapeutic agent (e.g., an inhibitor of a cancer-associated phosphatase, optionally, in combination with a second therapeutic agent) in an amount (e.g., an effective amount) and for a time sufficient to inhibit cancer cell growth, proliferation, metastasis, migration, or invasion. Cancer cell growth, proliferation, metastasis, migration, or invasion can be decreased in the subject or cancer cell culture at least 1%, 2%, 5%, 10%, 15%, 20%, 25%, 30%, 35%, 40%, 50%, 60%, 70%, 80%, 90%, 95% or more, compared to before the administration of the therapeutic agent (e.g., an inhibitor of a cancer-associated phosphatase, optionally, in combination with a second therapeutic agent). Cancer cell growth, proliferation, metastasis, migration, or invasion can be decreased in the subject or cancer cell culture between 5-20%, between 5-50%, between 10-50%, between 20-80%, or between 20-70%.

### Cancer types

In the methods described herein, the cancer or neoplasm may be any solid or liquid cancer and includes benign or malignant tumors, and hyperplasias, including gastrointestinal cancer (such as non-metastatic or metastatic colorectal cancer, pancreatic cancer, gastric cancer, esophageal cancer, hepatocellular cancer, cholangiocellular cancer, oral cancer, lip cancer); urogenital cancer (such as hormone sensitive or hormone refractory prostate cancer, renal cell cancer, bladder cancer, penile cancer); gynecological cancer (such as ovarian cancer, cervical cancer, endometrial cancer); lung cancer (such as small-cell lung cancer and non-small-cell lung cancer); head and neck cancer (e.g., head and neck squamous cell cancer); CNS cancer including malignant glioma, astrocytomas, retinoblastomas and brain metastases; malignant mesothelioma; non-metastatic or metastatic breast cancer (e.g., hormone refractory metastatic breast cancer); skin cancer (such as malignant melanoma, basal and squamous cell skin cancers, Merkel Cell Carcinoma, lymphoma of the skin, Kaposi Sarcoma); thyroid cancer; bone and soft tissue sarcoma; and hematologic neoplasias (such as multiple myeloma, acute myelogenous leukemia, chronic myelogenous leukemia, myelodysplastic syndrome, acute lymphoblastic leukemia, Hodgkin's lymphoma).

Additional cancers that can be treated according to the methods described herein include breast cancer, lung cancer, stomach cancer, colon cancer, liver cancer, renal cancer, colorectal cancer, prostate cancer, pancreatic cancer, cervical cancer, anal cancer, vulvar cancer, penile cancer, vaginal cancer, testicular cancer, pelvic cancer, thyroid cancer, uterine cancer, rectal cancer, brain cancer, head and neck cancer, esophageal cancer, bronchus cancer, gallbladder cancer, ovarian cancer, bladder cancer, oral cancer, oropharyngeal cancer, larynx cancer, biliary tract cancer, skin cancer, a cancer of the central nervous system, a cancer of the respiratory system, and a cancer of the urinary system. Examples of breast cancers include, but are not limited to, triple-negative breast cancer, triple-positive breast cancer, HER2-negative breast cancer, HER2-positive breast cancer, estrogen receptor-positive breast cancer, estrogen receptor-negative breast cancer, progesterone receptor-positive breast cancer, progesterone receptor-negative breast cancer, ductal carcinoma in situ (DCIS), invasive ductal carcinoma, invasive lobular carcinoma, inflammatory breast cancer, Paget disease of the nipple, and phyllodes tumor.

Other cancers that can be treated according to the methods described herein include leukemia (e.g., B-cell leukemia, T-cell leukemia, acute myeloid leukemia (AML), chronic myeloid leukemia (CML), acute lymphocytic (lymphoblastic) leukemia (ALL), chronic lymphocytic leukemia (CLL), and erythroleukemia), sarcoma (e.g., angiosarcoma, chondrosarcoma, Ewing's sarcoma, fibrosarcoma, gastrointestinal stromal tumor, leiomyosarcoma, liposarcoma, malignant peripheral nerve sheath tumor, malignant fibrous cytoma, osteosarcoma, pleomorphic sarcoma, rhabdomyosarcoma, synovial sarcoma, vascular sarcoma, Kaposi's sarcoma, dermatofibrosarcoma, epithelioid sarcoma, leyomyosarcoma, and neurofibrosarcoma), carcinoma (e.g., basal cell carcinoma, large cell carcinoma, small cell carcinoma, non-small cell lung carcinoma, renal carcinoma, hepatocarcinoma, gastric carcinoma, choriocarcinoma, adenocarcinoma, hepatocellular carcinoma, giant (or oat) cell carcinoma, squamous cell carcinoma, adenosquamous carcinoma, anaplastmic carcinoma, adrenocortical carcinoma, cholangiocarcinoma, Merkel cell carcinoma, ductal carcinoma in situ (DCIS), and invasive ductal carcinoma), blastoma (e.g., hepatoblastoma, medulloblastoma, nephroblastoma, neuroblastoma, pancreatoblastoma, pleuropulmonary blastoma, retinoblastoma, and glioblastoma multiforme), lymphoma (e.g., Hodgkin's lymphoma, non-Hodgkin's lymphoma, and Burkitt lymphoma), myeloma (e.g., multiple myeloma, plasmacytoma, localized myeloma, and extramedullary myeloma), melanoma (e.g., superficial spreading melanoma, nodular melanoma, lentigno maligna melanoma, acral lentiginous melanoma, and amelanotic melanoma), neuroma (e.g., ganglioneuroma, Pacinian neuroma, and acoustic neuroma), glioma (e.g., astrocytoma, oligoastrocytoma, ependymoma, brainstem glioma, optic nerve glioma, and oligoastrocytoma), pheochromocytoma, meningioma, malignant mesothelioma, and virally induced cancer.

The cancer may be highly innervated, metastatic, non-metastatic cancer, or benign (e.g., a benign tumor). The cancer may be a primary tumor or a metastasized tumor.

In some embodiments, the cancer is a cancer that is treated with immunotherapy (e.g., melanoma, non-small cell lung cancer, kidney cancer, renal cell carcinoma, bladder cancer, head and neck cancer, Hodgkin's lymphoma, leukemia, urothelial carcinoma, gastric cancer, microsatellite instability-high cancer, colorectal cancer, or hepatocellular carcinoma). In some embodiments, the cancer is a cancer for which immunotherapy is not effective (e.g., a cancer that cannot be treated using immunotherapy or a cancer that did not respond to treatment with immunotherapy).

Subjects who can be treated with the methods disclosed herein include subjects who have had one or more tumors resected, received chemotherapy or other pharmacological treatment for the cancer, received radiation therapy, and/or received other therapy for the cancer. Subjects who can be treated with the methods disclosed herein include subjects that do not respond to immunotherapy. Subjects who have not previously been treated for cancer can also be treated with the methods disclosed herein.

### Methods of treatment

### Administration

An effective amount of a therapeutic agent (e.g., an inhibitor of a cancer-associated phosphatase, optionally, in combination with a second therapeutic agent) described herein for treatment of cancer can be administered to a subject by standard methods. For example, the agent can be administered by any of a number of different routes including, e.g., intravenous, intradermal, subcutaneous, percutaneous injection, oral, transdermal (topical), or transmucosal. The therapeutic agent (e.g., an inhibitor of a cancer-associated phosphatase, optionally, in combination with a second therapeutic agent) can be administered orally or administered by injection, e.g., intramuscularly, or intravenously. The most suitable route for administration in any given case will depend on the particular agent administered, the patient, the particular disease or condition being treated, pharmaceutical formulation methods, administration methods (e.g., administration time and administration route), the patient's age, body weight, sex, severity of the diseases being treated, the patient's diet, and the patient's excretion rate. The agent can be encapsulated or injected, e.g., in a viscous form, for delivery to a chosen site, e.g., a tumor site. The agent can be provided in a matrix capable of delivering the agent to the chosen site. Matrices can provide slow release of the agent and provide proper presentation and appropriate environment for cellular infiltration. Matrices can be formed of materials presently in use for other implanted medical applications. The choice of matrix material is based on any one or more of: biocompatibility, biodegradability, mechanical properties, and cosmetic appearance and interface properties. One example is a collagen matrix.

The therapeutic agent (e.g., an inhibitor of a cancer-associated phosphatase, optionally, in combination with a second therapeutic agent) can be incorporated into pharmaceutical compositions suitable for administration to a subject, e.g., a human. Such compositions typically include the agent and a pharmaceutically acceptable carrier. As used herein the term "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances are known. Except insofar as any conventional media or agent is incompatible with the active compound, such media can be used in the described herein. Supplementary active compounds can also be incorporated into the compositions.

A pharmaceutical composition can be formulated to be compatible with its intended route of administration. Solutions or suspensions used for parenteral, intradermal, or subcutaneous application can include the following components: a sterile diluent such as water for injection, saline solution, fixed oils, polyethylene glycols, glycerin, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (e.g., a therapeutic agent described herein) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Systemic administration can also be by transmucosal or transdermal means. For transmucosal or transdermal administration, penetrants appropriate to the barrier to be permeated are used in the formulation. Such penetrants are generally known, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The active compounds can be prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. Liposomal suspensions (including liposomes targeted to infected cells with monoclonal antibodies to viral antigens) can also be used as pharmaceutically acceptable carriers. These can be prepared according to methods known to those skilled in the art.

Nucleic acid molecule agents described herein (e.g., therapeutic mRNAs) can be administered directly or inserted into vectors used as gene therapy vectors. Gene therapy vectors can be delivered to a subject by, for example, intravenous injection, local administration (see, e.g., U.S. Patent No. 5,328,470) or by stereotactic injection (see, e.g., Chen et al., PNAS 91:3054 1994). The pharmaceutical preparation of the gene therapy vector can include the gene therapy vector in an acceptable diluent, or can include a slow release matrix in which the gene delivery vehicle is embedded. Alternatively, where the complete gene delivery vector can be produced intact from recombinant cells, e.g., retroviral vectors, the pharmaceutical preparation can include one or more cells which produce the gene delivery system.

The pharmaceutical compositions can be included in a container, pack, or dispenser together with instructions for administration.

Methods of formulating pharmaceutical agents are known in the art, e.g., Niazi, Handbook of Pharmaceutical Manufacturing Formulations (Second Edition), CRC Press 2009, describes formulation development for liquid, sterile, compressed, semi-compressed and OTC forms. Transdermal and mucosal delivery, lymphatic system delivery, nanoparticles, controlled drug release systems, theranostics, protein and peptide drugs, and biologics delivery are described in Wang et al., Drug Delivery: Principles and Applications (Second Edition), Wiley 2016; formulation and delivery of peptide and protein agent is described, e.g., in Banga, Therapeutic Peptides and Proteins: Formulation, Processing, and Delivery Systems (Third Edition), CRC Press 2015.

### Combination therapies

A therapeutic agent described herein may be administered in combination with one or more additional therapies (e.g., 1, 2, 3 or more additional therapeutic agents). The two or more agents can be administered at the same time (e.g., administration of all agents occurs within 15 minutes, 10 minutes, 5 minutes, 2 minutes or less). The agents can also be administered simultaneously via co-formulation. The two or more agents can also be administered sequentially, such that the action of the two or more agents overlaps and their combined effect is such that the reduction in a symptom, or other parameter related to the disorder is greater than what would be observed with one agent or treatment delivered alone or in the absence of the other. The effect of the two or more treatments can be partially additive, wholly additive, or greater than additive (e.g., synergistic). Sequential or substantially simultaneous administration of each therapeutic agent can be effected by any appropriate route including, but not limited to, oral routes, intravenous routes, intramuscular routes, local routes, and direct absorption through mucous membrane tissues. The therapeutic agents can be administered by the same route or by different routes. For example, a first therapeutic agent of the combination may be administered by intravenous injection while a second therapeutic agent of the combination can be administered locally in a compound-impregnated microcassette. The first therapeutic agent may be administered immediately, up to 1 hour, up to 2 hours, up to 3 hours, up to 4 hours, up to 5 hours, up to 6 hours, up to 7 hours, up to, 8 hours, up to 9 hours, up to 10 hours, up to 11 hours, up to 12 hours, up to 13 hours, 14 hours, up to hours 16, up to 17 hours, up 18 hours, up to 19 hours up to 20 hours, up to 21 hours, up to 22 hours, up to 23 hours up to 24 hours or up to 1-7, 1-14, 1-21 or 1-30 days before or after the second therapeutic agent.

In other examples, an inhibitor of a cancer-associated phosphatase is administered in combination with a second therapeutic agent. The second therapeutic agent may be an immunomodulatory cytokine, an agent that inhibits myeloid derived suppressor cells, an immune checkpoint inhibitor, and anti-proliferative agent, a phosphoneoantigen vaccine, CAR-T, or any other therapeutic agent described herein.

### Dosing

Subjects that can be treated as described herein are subjects with cancer or at risk of developing cancer. The cancer may be a primary tumor or a metastasized tumor. In some embodiments, the cancer is a cancer associated with the expression or overexpression of a cancer-associated phosphatase. Subjects who can be treated with the methods disclosed herein include subjects who have had one or more tumors resected, received chemotherapy or other pharmacological treatment for the cancer, received radiation therapy, and/or received other therapy for the cancer. Subjects who have never previously been treated for cancer can also be treated using the methods described herein.

In some embodiments, the agent is administered in an amount and for a time effective to result in one of (or more, e.g., 2 or more, 3 or more, 4 or more of): (a) reduced tumor size, (b) reduced rate of tumor growth, (c) increased tumor cell death, (d) reduced tumor progression, (e) reduced number of metastases, (f) reduced rate of metastasis, (g) reduced tumor migration, (h) reduced tumor invasion, (i) reduced tumor volume, (j) decreased tumor recurrence, (k) increased survival of subject, or (l) increased progression free survival of subject.

The methods described herein may include a step of selecting a treatment for a patient. The method includes (a) identifying (e.g., diagnosing) a patient who has cancer or is at risk of developing cancer, and (b) selecting a therapeutic agent (e.g., an inhibitor of a cancer-associated phosphatase, optionally, in combination with a second therapeutic agent), to treat the condition in the patient. In some embodiments, the method includes administering the selected treatment to the subject. In some embodiments, a patient is identified as having cancer based on imaging (e.g., MRI, CT, or PET scan), biopsy, or blood sample (e.g., detection of blood antigen markers, circulating tumor DNA (e.g., by PCR). In some embodiments, a patient is identified as having cancer after presenting with one or more symptoms of a paraneoplastic syndrome (e.g., fever, auto-antibodies directed against nervous system proteins, ataxia, dizziness, nystagmus, difficulty swallowing, loss of muscle tone, loss of fine motor coordination, slurred speech memory loss, vision loss, sleep disturbances, dementia, seizures, dysgeusia, cachexia, anemia, itching, or sensory loss in the limbs). In some embodiments, a patient presents with symptoms of paraneoplastic syndrome and is then identified as having cancer based on imaging (e.g., CT, MRI, or PET scans).

In one example, the method includes (a) identifying (e.g., diagnosing) a patient who has cancer, (b) optionally evaluating the subject (e.g., the cancer cells of the subject) for expression or overexpression of a cancer-associated phosphatase, and (c) selecting a therapeutic agent (e.g., an inhibitor of a cancer-associated phosphatase, optionally, in combination with a second therapeutic agent) to treat the patient if a cancer cell exhibits expression or overexpression of the cancer-associated phosphatase. In some embodiments, the method includes administering the selected treatment to the subject.

The method may also include a step of assessing the subject for a parameter of cancer progression or remission, e.g., assessing the subject for one or more (e.g., 2 or more, 3 or more, 4 or more) of: primary tumor size (e.g., by imaging), number of metastases (e.g., by imaging or biopsy), cell death in situ (e.g., by biopsy), blood antigen markers (e.g., by ELISA), circulating tumor DNA (e.g., by PCR), or function of the affected organ (e.g., by a test of circulating enzymes for liver, albuminuria for kidney, lung capacity for lung, etc.).

The methods described herein may also include a step of assessing the subject for a parameter of immune response, e.g., assessing the subject for one or more (e.g., 2 or more, 3 or more, 4 or more) of: Th2 cells, T cells, circulating monocytes, neutrophils, peripheral blood hematopoietic stem cells, macrophages, mast cell degranulation, activated B cells, NKT cells, macrophage phagocytosis, macrophage polarization, antigen presentation, immune cell activation, immune cell proliferation, immune cell lymph node homing or egress, T cell differentiation, immune cell recruitment, immune cell migration, lymph node innervation, dendritic cell maturation, HEV development, TLO development, or cytokine production. In embodiments, the method includes measuring a cytokine or marker associated with the particular immune cell type. In some embodiments, the method includes measuring a chemokine, receptor, or immune cell trafficking molecule (e.g., performing an assay to measure the chemokine, marker, or receptor). The assessing may be performed after the administration, before the first administration and/or during a course a treatment, e.g., after a first, second, third, fourth or later administration, or periodically over a course of treatment, e.g., once a month, or once every 3 months. In some embodiments, the method includes assessing the subject prior to treatment or first administration and using the results of the assessment to select a subject for treatment. In certain embodiments, the method also includes modifying the administering step (e.g., stopping the administration, increasing or decreasing the periodicity of administration, increasing or decreasing the dose of the therapeutic agent) based on the results of the assessment.

In other examples, immune effects (e.g., immune cell activities) are modulated in a subject (e.g., a subject having a cancer or inflammatory or autoimmune condition) or in a cultured cell by at least 1%, 2%, 5%, 10%, 15%, 20%, 25%, 30%, 35%, 40%, 50%, 60%, 70%, 80%, compared to before an administration, e.g., of a dosing regimen, of a therapeutic agent (e.g., an inhibitor of a cancer-associated phosphatase, optionally, in combination with a second therapeutic agent) such as those described herein. In certain embodiments, the immune effects are modulated in the subject or a cultured cell between 5-20%, between 5-50%, between 10-50%, between 20-80%, between 20-70%, between 50-100%, or between 100-500%. The immune effects described herein may be assessed by standard methods:

The therapeutic agent (e.g., an inhibitor of a cancer-associated phosphatase, optionally, in combination with a second therapeutic agent) described herein are administered in an amount (e.g., an effective amount) and for a time sufficient to effect one of the outcomes described above. The therapeutic agent (e.g., an inhibitor of a cancer-associated phosphatase, optionally, in combination with a second therapeutic agent) may be administered once or more than once. The therapeutic agent (e.g., an inhibitor of a cancer-associated phosphatase, optionally, in combination with a second therapeutic agent) may be administered once daily, twice daily, three times daily, once every two days, once weekly, twice weekly, three times weekly, once biweekly, once monthly, once bimonthly, twice a year, or once yearly. Treatment may be discrete (e.g., an injection) or continuous (e.g., treatment via an implant or infusion pump). Subjects may be evaluated for treatment efficacy 1 week, 2 weeks, 1 month, 2 months, 3 months, 4 months, 5 months, 6 months or more following administration of a therapeutic agent (e.g., an inhibitor of a cancer-associated phosphatase, optionally, in combination with a second therapeutic agent) depending on therapeutic agent and route of administration used for treatment. Depending on the outcome of the evaluation, treatment may be continued or ceased, treatment frequency or dosage may change, or the patient may be treated with a different therapeutic agent. Subjects may be treated for a discrete period of time (e.g., 1, 2, 3, 4, 5, 6, 7, 8, 9, 10, 11, or 12 months) or until the disease or condition is alleviated, or treatment may be chronic depending on the severity and nature of the disease or condition being treated.

### Examples

The following examples are put forth so as to provide those of ordinary skill in the art with a description of how the compositions and methods described herein may be used, made, and evaluated, and are intended to be purely exemplary of the invention and are not intended to limit the scope of what the inventors regard as their invention.

### Example 1. Expression of ALPP and ALPPL2 in lymphoblastoid cell lines

Epstein-Barr virus (EBV) is a ubiquitous human herpesvirus that infects healthy B-cells within us. The virus is known to transform infected B-cells to become cancerous. However, these transformed B-cells are continually removed by T-cells such that for the vast majority of subjects, EBV is a life-long asymptomatic disease. For subjects where T-cells are not functioning, such as patient with primary immunodeficiency, or those on powerful immunosuppressive agents (e.g., transplant patients), T-cells are prevented from fighting these cancers. In these patients the EBV-infected B-cells grow uncontrollably and manifest as an aggressive type of lymphoma termed post-transplant lymphoproliferative disease (PTLD).

PTLD-like tumors were generated by infecting B-cells from healthy individuals. This infection resulted in the formation of lymphoblastoid cell lines (LCLs) which grow indefinitely. When healthy donor's T-cells were added, the T-cell kill the LCLs. Thus this system may be used as a model of cancer.

LCLs express a large number of phosphopeptides, yet they do not express ALPP or ALPP-L2. Culturing the LCLs in phosphatase rich media, strips the surface HLA-molecules of phosphopeptides and reduces recognition of LCLs by autologous T-cells by 50%.

Upon expression of ALPP and ALPPL2 in LCLs, autologous T-cells are less able to recognize phosphatase-expressing LCLs.

### Epstein-Barr virus (EBV)-lymphoblastoid cell line (LCL) generation

Peripheral blood mononuclear cells (PBMCs) were counted and suspended at 1×10⁷ cells/mL in supplemented RPMI. EBV stock was thawed and cyclosporin A was added to the EBV stock solution to a final concentration of 800 ng/mL. 1 mL of PBMCs and 1 mL of EBV stock + cyclosporin A was added per well in a 24 well plate. The final number of PBMCs was 1×10⁷ cells per well and the final concentration of cyclosporin A was 400 ng/mL. The plate was incubated for three weeks at 37°C and 5% CO2. The cells were counted and cultured per standard methods of LCL cell culture known to one of skill in the art.

### Lentivector cloning

Using Gateway method, either ALPP, ALPPL2 or Luciferase cDNA sequence were cloned into pLV lenti-backbone (VectorBuilder). For identifying gene expressions, either GFP or mCherry was used as marker under the regulation of a CMV promoter. Detailed vector designs are provided in Fig. 5.

### Lentivirus production

On day -1, HEK 293FT packaging cells were plated in 15cm dishes at ~40% confluency. On day 0, a cell density between 80%-90% was achieved before transfection. Mirus-TransIT^{™} transfection reagent was used to transfect HEK293 cells with ALPP / ALPPL2 / GL lentivector and packaging vectors pSPAX2 / pMD2.G. On day 2, supernatant was collected and passed through a 0.2µM filter. The virus was concentrated by ultracentrifugation at 120,000g, 2h, 4°C. The pellet was resuspended with 200µl PBS. Resuspended virus was aliquoted and stored at -80°C for future use. Virus was titrated by using HEK293 cells and tested by flow cytometry.

### Transduction of LCL cells to make ALPP/ALPPL2 overexpressing lines

Virus was added into LCL cells at M.O.I = 100, in the presence of 4µg/ml polybrene. Four hours later, the media for LCL cells was changed. On day 3, ALPP/ALPPL2/Luciferase-overexpressing cells were purified by FACS sort, depending on GFP/mCherry expressions. GFP/mCherry-positive cells were cultured and thought as ALPP/ALPPL2/Luciferase-overexpressing lines (LCL-ALPP, LCL-ALPPL2, LCL-GL) (Fig. 6).

### Generation of LCL-specific CD8+ T-cell line

In 24 well plates, autologous PBMC were seeded together with LCL at ratio, PBMC:LCL=40:1 (2e6: 5e4) in 2ml RPMI media. After 10 days, the responder cells were re-stimulated weekly with irradiated (40 Gy) LCLs at a responder-to-stimulator ratio of 4:1 (1×10⁶: 2.5×10⁵). Two weekly doses of rhlL-2 (50-100 IU/mL) were added from day 14.

### Impact of ALPPIALPPL2 on specific CD8⁺ T-cell killing of autologous LCL

Parent LCLs were pre-labeled with Violet dye (Thermofisher, CellTrace^{™} Violet Cell Proliferation Kit, C34557). Violet dye-labeled parent LCLs were seeded together with LCL-ALPP, LCL-ALPPL2 or LCL-GL (at 1:1 ratio) in the presence (CD8:LCLs=10:1) or absence (CD8:LCLs=0:1) of autologous LCL-specific CD8 T cells in 96 well plate. After 16 hours, cells were harvested and checked by FACS. Both ALPP and ALPPL2 expressions negatively regulated autologous CD8 T cell killing efficacy. No obvious killing preference between parent LCL and LCL-GL cells was observed (Figs. 7A-B).

### Example 2. ALPP/ALPPL2 inhibitor, ML095, recovers specific CD8⁺T-cell killing against tumor cells

### Impacts of ALPP/ALPPL2 on specific CD8⁺ T-cell killing of haploidentical targets

To generated tumor specific CD8-T cell lines, two haploidentical tumor cell lines and HLA-matched human PBMCs were adopted. Tumor cell lines are shown in Table 2.

**Table 2.**

| **Cell line** | **HLA type** | **Cancer type** | **ALPP/ALPPL2 expressions** |
|---|---|---|---|
| H2009 | A*03:01/B*07:02/C*07:02 | Human lung cancer | Low |
| | A*03:01/B*07:02/C*07:02 | | |
| A431 | A*03:01/B*07:02/C*07:02 | Human epidermoid carcinoma | High |
| | A*03:01/B*07:02/C*07:02 | | |

### Transduction of H2009 to make ALPP/ALPPL2 overexpressing lines

Virus was added into H2009 at M.O.I = 100, in the presence of 4ug/ml polybrene. Four hours later, the media for H2009 was changed. On day 3, ALPP/ALPPL2-overexpressing cells were purified by FACS sort, depending on GFP/mCherry expressions. GFP/mCherry-positive cells were cultured and thought as ALPP/ALPPL2-overexpressing lines (H2009ALPP OE, H2009ALPPL2 OE). ALPP or ALPPL2 expression was verified by Western-blot (Fig. 8A) and NBT BCIP Reaction assay (Fig. 9A).

### Transduction of A431 to knock-out ALPPIALPPL2 in A431 using CRISPR-Cas9

Lentivector LentiCRISPR-GFP was used to introduce both Cas9 and gRNA, targeting either ALPP, ALPPL2 or irrelevant target (scramble), into A431. Three days later, GFP+ cells were sorted and seeded into 96 well plate at concentration 0.3 cells/well for single cell cloning of ALPP or ALPPL2 knock-out lines (A431ALPP KO, A431ALPPL2 KO). Once cells were growing, the knock-out efficiency for both ALPP and ALPPL2 were verified by Western-blot (Fig. 8B) and NBT-BCIP Reaction assay (Fig. 9B).

### NBT-BCIP Reaction assay for ALPPIALPPL2

The cells were fixed in ice-cold acetone-methanol mixture (30:70) for 5 min. The cells were washed two times with PBS and endogenous heat-labile alkaline phosphatases were inactivated by incubation in TMN substrate buffer (0.1M Tris-HCl, pH 9.5 containing 0.1M NaCl and 5 mM MgCl2) at 60°C for 30 min. Substrate buffer was discarded and staining for heat-stable PLAP was performed by using fresh TMN buffer containing 0.175 mg/mL of 5-bromo-4-chloro-3-indolyl phosphate (BCIP; Sigma-Aldrich) and 0.45 mg/mL of nitrotetrazolium blue chloride (NBT; Sigma-Aldrich). The staining was performed at RT for at least 3 h. Samples were washed two times with PBS and observed under an inverted light microscope (Figs. 9A-B).

### Generation of haploidentical targets-specific CD8⁺ T-cell line

In 24 well plates, HLA-matching PBMC (A*03:01/B*07:02/C*07:02) were seeded together with either H2009 or A431 at ratio PBMC: tumor cells=40:1 (2×10⁶: 5×10⁴) in 2ml RPMI media. Both H2009 and A431 were irradiated (40 Gy) before use. Starting on day 10, the responder cells were re-stimulated weekly with irradiated (40 Gy) H2009 or A431 at a responder-to-stimulator ratio of 4:1 (1×10⁶: 2.5×10⁵). Two weekly doses of rhlL-2 (50-100 IU/mL) were added from day 14.

### Impact of ALPP/ALPPL2 on specific CD8⁺ T-cell killing of HLA-matched haploidentical targets

Parental cell lines (H2009 or A431) were pre-labeled with Violet dye (Thermofisher, CellTracer^{™} Violet Cell Proliferation Kit C34557). Violet dye-labeled parental cell lines were seeded together with lentiviral engineered cell lines (at 1:1 ratio) in the presence (CD8: tumor cells =1:1, 10:1) or absence (CD8: tumor cells=0:1) of autologous specific CD8-T cells in 96 well plates. After 16 hours, cells were harvested and checked by FACS.

Results show that both ALPP/ALPPL2 expressions negatively regulated specific CD8⁺ T-cell killing efficacy. Overexpression of either ALPP or ALPPL2 helps tumor cells escape from specific CD8⁺ T-cell killing (Fig. 10A, H2009 results). Knock-out of either ALPP or ALPPL2 increases the sensitivity of tumor cells to specific CD8⁺ T-cell killing (Fig. 10B, A431 results). No killing preference between parental A431 and A431 scramble KO line was observed.

### Impact of ALPP/ALPPL2 inhibitor, ML095, on specific CD8⁺ T-cell killing of HLA-matched haploidentical targets

Parental cell lines (H2009 or A431) were pre-labeled with Violet dye (Thermofisher, CellTracer^{™} Violet Cell Proliferation Kit, C34557). Violet dye-labeled parental cell lines were seeded together with lentiviral engineered cell lines (at 1:1 ratio) in the presence (CD8: tumor cells = 10:1) or absence (CD8: tumor cells= 0:1) of autologous specific CD8⁺ T-cells in 96 well plates. ML095 was added at concentration 0uM, 2uM and 10uM. After 16 hours, cells were harvested and checked by FACS.

Results show that adding ML095 recovers specific CD8⁺ T-cell killing against H2009^{ALPP OE} (Figs. 11A-B) and H2009^{ALPPL2 OE} (Figs. 12A-B). Results show that adding ML095 increases specific CD8⁺ T-cell killing of A431 as compared to A431^{ALPP KO} (Figs. 13A-B) or A431^{ALPPL2 KO} (Figs. 14A-B), without showing any impacts on A431^{scramble KO} (Figs. 15A-B).

### Other Embodiments

While the invention has been described in connection with specific embodiments thereof, it will be understood that it is capable of further modifications and this application is intended to cover any variations, uses, or adaptations of the invention following, in general, the principles of the invention and including such departures from the invention that come within known or customary practice within the art to which the invention pertains and may be applied to the essential features hereinbefore set forth, and follows in the scope of the claims. Other embodiments are within the claims.

### Embodiments

1. A method of increasing expression of a phosphoneoantigen in a cancer cell, said method comprising the step of contacting said cancer cell with a therapeutic agent that decreases the activity of a cancer-associated phosphatase in said cancer cell.
2. The method of embodiment 1, wherein said increasing expression of said phosphoneoantigen of a cancer cell restores expression of said phosphoneoantigen on the surface of said cancer cell.
3. The method of embodiment 1, wherein said cancer-associated phosphatase is an alkaline phosphatase.
4. The method of embodiment 3, wherein said alkaline phosphatase is selected from placental alkaline phosphatase (ALPP), placental-like alkaline phosphatase (ALPPL2), intestinal alkaline phosphatase (ALPI), or tissue-nonspecific alkaline phosphatase (ALPL).
5. The method of embodiment 1, wherein said cancer-associated phosphatase is an acid phosphatase.
6. The method of embodiment 5, wherein said acid phosphatase is prostatic acid phosphatase (ACPP).
7. The method of embodiment 1, wherein said cancer-associated phosphatase is a protein serine/threonine phosphatase (PSP).
8. The method of any one of embodiments 1-7, wherein said therapeutic agent is an inhibitor of said cancer-associated phosphatase.
9. The method of embodiment 8, wherein said inhibitor of said cancer-associated phosphatase is ML095, thiophosphate, L-p-bromotetramisole, tetramisole, levamisole, 5804079, L-phenylalanine, 1-paphthyl phosphate, MLS-0315848, MLS0390945, or MLS-0315687.
10. The method of any one of embodiments 1-9, wherein said method further comprises contacting said cancer cell with an immune checkpoint modulator.
11. The method of embodiment 10, wherein said immune checkpoint modulator is an anti-PD-1 antibody, an anti-CTLA-4 antibody, an anti-TIM-3 antibody, an anti-NKG2A antibody, an anti-LAG-3 antibody, an anti-cMet antibody, an anti-CTLA-4/PD-1 bispecific antibody, an anti-TIM-3/PD-1 bispecific antibody, an anti-PD1/LAG3 bispecific antibody, an anti-PD-1/cMet bispecific antibody, or an antigen-binding fragment thereof.
12. The method of embodiment 10, wherein said immune checkpoint modulator is pembrolizumab, nivolumab, atelizumab, spartalizumab, camrelizumab, pidilizumab, cemiplimab, tislelizumab, JS001, MEDI0680, BCD-100, JNJ-63723283, CBT-501, TSR-042, MGA012, PF-06801591, KN035, LZM009, XmAb20717, AGEN2034, Sym021, AK105, AK104, HLX10, CS1003, STI-1110, MGD013, MCLA-134, AM001, or EDI5752.
13. The method of any one of embodiments 1-12, wherein said method further comprises contacting said cancer cell with an immunomodulatory cytokine.
14. The method of embodiment 13, wherein said immunomodulatory cytokine is selected from IL-2, IL-12, IL-15, Neo-2/15, IFNα, IFNβ, and IFNγ.
15. The method of any one of embodiments 1-14, wherein said method further comprises contacting said cancer cell with an agent that inhibits myeloid derived suppressor cells.
16. The method of embodiment 15, wherein the agent that inhibits myeloid derived suppressor cells is selected from an indoleamine 2,3-dioxygenase (IDO) inhibitor, an anti-VEGF antibody, a nitric oxide synthetase (NOS) inhibitor, a CSF-1R inhibitor, an arginase inhibitor, or a multi-kinase inhibitor.
17. The method of any one of embodiments 1-16 wherein said cancer cell is in a subject having cancer, and wherein said method further comprises vaccinating said subject with said phosphoneoantigen and a suitable adjuvant.
18. The method of any one of embodiments 1-16 wherein said cancer cell is in a subject having cancer, and wherein said method further comprises administering to said subject a T-cell that has been genetically modified to express a T-cell receptor (TCR), wherein said TCR binds specifically to a phosphoneoantigen.
19. The method of any one of embodiments 1-18, wherein said method further comprises contacting said cancer cell with an anti-proliferative agent.
20. The method of embodiment 19, wherein said anti-proliferative agent is selected from MK-2206, ON 013105, RTA 402, BI 2536, Sorafenib, ISIS-STAT3Rx, a microtubule inhibitor, a topoisomerase inhibitor, a platin, an alkylating agent, an anti-metabolite, paclitaxel, gemcitabine, doxorubicin, vinblastine, etoposide, 5-fluorouracil, carboplatin, altretamine, aminoglutethimide, amsacrine, anastrozole, azacitidine, bleomycin, busulfan, carmustine, chlorambucil, 2-chlorodeoxyadenosine, cisplatin, colchicine, cyclophosphamide, cytarabine, cytoxan, dacarbazine, dactinomycin, daunorubicin, docetaxel, estramustine phosphate, floxuridine, fludarabine, gentuzumab, hexamethylmelamine, hydroxyurea, ifosfamide, imatinib, interferon, irinotecan, lomustine, mechlorethamine, melphalan, 6-mercaptopurine, methotrexate, mitomycin, mitotane, mitoxantrone, pentostatin, procarbazine, rituximab, streptozocin, tamoxifen, temozolomide, teniposide, 6-thioguanine, topotecan, trastuzumab, vincristine, vindesine, or vinorelbine.
21. The method of any one of embodiments 1-20, wherein said cancer cell has been previously determined to have increased activity of said cancer-associated phosphatase.
22. A method of treating or reducing the probability of occurrence of a cancer in a subject in need thereof, wherein said method comprises administering to said subject a therapeutic agent that decreases the activity of a cancer-associated phosphatase in the cells of said cancer.
23. The method of embodiment 22, wherein said therapeutic agent increases expression of a phosphoneoantigen in the cells of said cancer.
24. The method of embodiment 22, wherein said subject has been previously determined to have increased activity of said cancer-associated phosphatase in the cells of said cancer.
25. The method of embodiment 22, wherein said cancer-associated phosphatase is an alkaline phosphatase.
26. The method of embodiment 25, wherein said alkaline phosphatase is selected from placental alkaline phosphatase (ALPP), placental-like alkaline phosphatase (ALPPL2), intestinal alkaline phosphatase (ALPI), or tissue-nonspecific alkaline phosphatase (ALPL).
27. The method of embodiment 22, wherein said cancer-associated phosphatase is an acid phosphatase.
28. The method of embodiment 27, wherein said acid phosphatase is prostatic acid phosphatase (ACPP).
29. The method of embodiment 22, wherein said cancer-associated phosphatase is a protein serine/threonine phosphatase (PSP).
30. The method of any one of embodiments 22-29, wherein said therapeutic agent is an inhibitor of said cancer-associated phosphatase.
31. The method of embodiment 30, wherein said inhibitor of said cancer-associated phosphatase is selected from ML095, thiophosphate, L-p-bromotetramisole, tetramisole, levamisole, 5804079, L-phenylalanine, 1-paphthyl phosphate, MLS-0315848, MLS0390945, or MLS-0315687.
32. The method of any one of embodiments 22-31, wherein said method further comprises administering an immune checkpoint modulator to said subject.
33. The method of embodiment 32, wherein said immune checkpoint modulator is an anti-PD-1 antibody, an anti-CTLA-4 antibody, an anti-TIM-3 antibody, an anti-NKG2A antibody, an anti-LAG-3 antibody, an anti-cMet antibody, an anti-CTLA-4/PD-1 bispecific antibody, an anti-TIM-3/PD-1 bispecific antibody, an anti-PD1/LAG3 bispecific antibody, an anti-PD-1/cMet bispecific antibody, or an antigen-binding fragment thereof.
34. The method of embodiment 32, wherein said immune checkpoint modulator is pembrolizumab, nivolumab, atelizumab, spartalizumab, camrelizumab, pidilizumab, cemiplimab, tislelizumab, JS001, MEDI0680, BCD-100, JNJ-63723283, CBT-501, TSR-042, MGA012, PF-06801591, KN035, LZM009, XmAb20717, AGEN2034, Sym021, AK105, AK104, HLX10, CS1003, STI-1110, MGD013, MCLA-134, AM001, or EDI5752.
35. The method of any one of embodiments 22-34, wherein said method further comprises administering to said subject an immunomodulatory cytokine.
36. The method of embodiment 35, wherein said immunomodulatory cytokine is selected from IL-2, IL-12, IL-15, Neo-2/15, IFNα, IFNβ, and IFNγ.
37. The method of any one of embodiments 22-36, wherein said method further comprises contacting said cancer cell with an agent that inhibits myeloid derived suppressor cells.
38. The method of embodiment 37, wherein the agent that inhibits myeloid derived suppressor cells is selected from an indoleamine 2,3-dioxygenase (IDO) inhibitor, an anti-VEGF antibody, a nitric oxide synthetase (NOS) inhibitor, a CSF-1R inhibitor, an arginase inhibitor, or a multi-kinase inhibitor.
39. The method of any one of embodiment 22-38, wherein said method further comprises vaccinating said subject with a phosphoneoantigen and a suitable adjuvant.
40. The method of any one of embodiments 22-38, wherein said method further comprises administering to said subject a T-cell that has been genetically modified to express a TCR, wherein said TCR binds specifically to a phosphoneoantigen.
41. The method of any one of embodiments 22-40, wherein said method further comprises administering to said subject an anti-proliferative agent.
42. The method of embodiment 41, wherein said anti-proliferative agent is selected from MK-2206, ON 013105, RTA 402, BI 2536, Sorafenib, ISIS-STAT3Rx, a microtubule inhibitor, a topoisomerase inhibitor, a platin, an alkylating agent, an anti-metabolite, paclitaxel, gemcitabine, doxorubicin, vinblastine, etoposide, 5-fluorouracil, carboplatin, altretamine, aminoglutethimide, amsacrine, anastrozole, azacitidine, bleomycin, busulfan, carmustine, chlorambucil, 2-chlorodeoxyadenosine, cisplatin, colchicine, cyclophosphamide, cytarabine, cytoxan, dacarbazine, dactinomycin, daunorubicin, docetaxel, estramustine phosphate, floxuridine, fludarabine, gentuzumab, hexamethylmelamine, hydroxyurea, ifosfamide, imatinib, interferon, irinotecan, lomustine, mechlorethamine, melphalan, 6-mercaptopurine, methotrexate, mitomycin, mitotane, mitoxantrone, pentostatin, procarbazine, rituximab, streptozocin, tamoxifen, temozolomide, teniposide, 6-thioguanine, topotecan, trastuzumab, vincristine, vindesine, or vinorelbine.
43. The method of any one of embodiments 1-42, wherein said cancer is selected from acute leukemia, acute lymphocytic leukemia, acute myelocytic leukemia, acute myeloblastic leukemia, acute promyelocytic leukemia, acute myelomonocytic leukemia, acute monocytic leukemia, acute erythroleukemia, chronic leukemia, chronic myelocytic leukemia, chronic lymphocytic leukemia, Hodgkin's disease, non-Hodgkin's disease, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, uterine cancer, testicular cancer, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodenroglioma, schwannoma, meningioma, melanoma, neuroblastoma, or retinoblastoma.
44. A method of identifying a subject at risk of developing cancer, wherein said method comprises determining the activity of a cancer-associated phosphatase in a cell of said subject, wherein increased activity of said cancer-associated phosphatase indicates an increased risk of developing said cancer.
45. The method of embodiment 44, wherein said cancer-associated phosphatase is an alkaline phosphatase.
46. The method of embodiment 45, wherein said alkaline phosphatase is selected from placental alkaline phosphatase (ALPP), placental-like alkaline phosphatase (ALPPL2), intestinal alkaline phosphatase (ALPI), tissue-nonspecific alkaline phosphatase (ALPL).
47. The method of embodiment 44, wherein said cancer-associated phosphatase is an acid phosphatase.
48. The method of embodiment 47, wherein said acid phosphatase is prostatic acid phosphatase (ACPP).
49. The method of embodiment 48, wherein said cancer-associated phosphatase is a protein serine/threonine phosphatase (PSP).
50. The method of any one of embodiments 44-49, wherein if said subject is determined to have an increased risk of developing cancer, said subject is administered a therapeutic agent that decreases the activity of said cancer-associated phosphatase in the cells of said cancer.
51. The method of embodiment 50, wherein said therapeutic agent is an inhibitor of said cancer-associated phosphatase.
52. The method of embodiment 51, wherein said inhibitor of said cancer-associated phosphatase is selected from ML095, thiophosphate, L-p-bromotetramisole, tetramisole, levamisole, 5804079, L-phenylalanine, 1-paphthyl phosphate, MLS-0315848, MLS0390945, or MLS-0315687.
53. The method of any one of embodiments 44-52, wherein if said subject is determined to have an increased risk of developing said cancer, said subject is vaccinated with a phosphoneoantigen and a suitable adjuvant.
54. The method of embodiment 53, wherein an increase in said phosphoneoantigen is associated with increased activity of said cancer-associated phosphatase.
55. A method of producing a nucleic acid encoding a T cell receptor (TCR) that binds to a phosphoneoantigen, the method comprising
   (a) immunizing a mammal, except for a human, with a phosphoneoantigen, wherein the immunizing optionally further comprises administering an adjuvant;
   (b) isolating a cell from the mammal of step (a) that expresses a TCR that binds to the phosphoneoantigen antigen; and
   (c) isolating a nucleic acid from the cell of step (b) that encodes the TCR that binds to the phosphoneoantigen antigen.
56. The method of embodiment 55, further comprising expressing the nucleic acid encoding the TCR that binds to the phosphoneoantigen in a host cell, thereby producing a TCR that binds to said phosphoneoantigen.
57. A method of treating a cancer in a subject in need thereof, wherein said method comprises administering to said subject a T-cell that has been genetically modified to express a TCR, wherein said TCR binds specifically to a phosphoneoantigen.
58. The method of any one of embodiments 55-57, wherein an increase in said phosphoneoantigen is associated with increased activity of a cancer-associated phosphatase.
59. The method of embodiment 58, wherein said cancer-associated phosphatase is an alkaline phosphatase.
60. The method of embodiment 59, wherein said alkaline phosphatase is selected from placental alkaline phosphatase (ALPP), placental-like alkaline phosphatase (ALPPL2), intestinal alkaline phosphatase (ALPI), tissue-nonspecific alkaline phosphatase (ALPL).
61. The method of embodiment 58, wherein said cancer-associated phosphatase is an acid phosphatase.
62. The method of embodiment 61, wherein said acid phosphatase is prostatic acid phosphatase (ACPP).
63. The method of embodiment 58, wherein said cancer-associated phosphatase is a protein serine/threonine phosphatase (PSP).
64. A method of treating or reducing the probability of occurrence of a cancer in a subject in need thereof, wherein said method comprises vaccinating said subject with a phosphoneoantigen and a suitable adjuvant.
65. The method of embodiment 64, wherein an increase in said phosphoneoantigen is associated increased activity of a cancer-associated phosphatase.
66. The method of embodiment 64, wherein said cancer-associated phosphatase is an alkaline phosphatase.
67. The method of embodiment 66, wherein said alkaline phosphatase is selected from placental alkaline phosphatase (ALPP), placental-like alkaline phosphatase (ALPPL2), intestinal alkaline phosphatase (ALPI), or tissue-nonspecific alkaline phosphatase (ALPL).
68. The method of embodiment 64, wherein said cancer-associated phosphatase is an acid phosphatase.
69. The method of embodiment 68, wherein said acid phosphatase is prostatic acid phosphatase (ACPP).
70. The method of embodiment 64, wherein said cancer-associated phosphatase is a protein serine/threonine phosphatase (PSP).

## Claims

1. A method of identifying an inhibitor of a placental alkaline phosphatase comprising
identifying a cancer cell comprising an increased expression of a placental alkaline phosphatase selected from alkaline phosphatase placental type (ALPP) or alkaline phosphatase placental-like 2 (ALPP-L2),
contacting said cancer cell with a potential therapeutic candidate, and
measuring the expression of a phosphoneoantigen, wherein an increase in the expression of said phosphoneoantigen is correlated to said therapeutic candidate being an inhibitor of ALPP or ALPP-L2.

2. The method of claim 1, wherein said placental alkaline phosphatase is ALPP.

3. The method of claim 1, wherein said placental alkaline phosphatase is ALPP-L2.

4. The method of claim 1, wherein said inhibitor of said cancer-associated phosphatase is ML095, thiophosphate, L-p-bromotetramisole, tetramisole, levamisole, 5804079, L-phenylalanine, 1-paphthyl phosphate, MLS-0315848, MLS0390945, or MLS-0315687.

5. The method of claim 1, wherein the inhibitor is a nucleic acid molecule, a polypeptide, a nuclease, or a small molecule.

6. The method of claim 5, wherein the polypeptide is an antibody molecule.

7. The method of claim 1, wherein the cancer cell is identified by measuring the enzymatic activity of the phosphatase or by measuring the level of expression of the phosphatase.

8. The method of claim 1, wherein the cancer cell has an increased phosphatase activity of 1%, 2%, 5%, 10%, 20%, 30%, 40%, 50%, 60%, 70%, 80%, 90%, 100%, 150%, 200%, 500%, or 1000% or greater relative to a wild-type cell of the same type.

9. The method of claim 8, wherein:
(a) the cancer cell has an increased phosphatase activity of 20% relative to a wild-type cell of the same type; and/or
(b) the cancer cell has an increased phosphatase activity of 50% relative to a wild-type cell of the same type.

10. The method of claim 1, wherein the expression level of the phosphoneoantigen increases by at least 1%, 2%, 5%, 10%, 20%, 30%, 40%, 50%, 60%, 70%, 80%, 90%, 100%, 150%, 200%, 500%, or 1000%.

11. The method of claim 10, wherein the expression level of the phosphoneoantigen increases by at least 20% after contact with the therapeutic agent.

12. The method of claim 10, wherein the expression level of the phosphoneoantigen increases by at least 50% after contact with the therapeutic agent.

13. The method of claim 1, wherein the cancer cell is isolated from a patient with gastrointestinal cancer, urogenital cancer, gynecological cancer, lung cancer, head and neck cancer, cancer of the central nervous system, malignant mesothelioma, breast cancer, skin cancer, thyroid cancer, bone and soft tissue sarcoma, hematologic neoplasias, stomach cancer, colon cancer, liver cancer, renal cancer, colorectal cancer, prostate cancer, pancreatic cancer, cervical cancer, anal cancer, vulvar cancer, penile cancer, vaginal cancer, testicular cancer, pelvic cancer, thyroid cancer, uterine cancer, rectal cancer, brain cancer, head and neck cancer, esophageal cancer, bronchus cancer, gallbladder cancer, ovarian cancer, bladder cancer, oral cancer, oropharyngeal cancer, larynx cancer, biliary tract cancer, a cancer of the respiratory system, or a cancer of the urinary system.

14. A method of increasing expression of a phosphoneoantigen in a cancer cell, said method comprising the step of contacting said cancer cell with a therapeutic agent that decreases the activity of a cancer-associated phosphatase in said cancer cell.

15. A therapeutic agent for use in a method of increasing expression of a phosphoneoantigen in a cancer cell, said method comprising the step of contacting said cancer cell with the therapeutic agent, wherein the therapeutic agent decreases the activity of a cancer-associated phosphatase in said cancer cell, further wherein said therapeutic agent is an inhibitor of said cancer-associated phosphatase.
